# EUROPEAN PATENT SPECIFICATION

(11) **EP 4 087 650 B1**
(45) Date of publication and mention of the grant of the patent: **02.12.2026**
(21) Application number: 20711668.2
(22) Date of filing: 10.01.2020
(51) Int. Cl.: A61P 7/00, A61P 9/00, A61P 35/00, C07D 203/24, C07D 401/12, C07D 405/12, A61K 31/396

(54) **AROMATIC SULPHONAMIDES DERIVATIVES THAT INHIBITS PDI A3, THEIR SYNTHESIS AND USE**
AROMATISCHE SULFONAMIDDERIVATE, DIE PDI A3 HEMMEN, IHRE SYNTHESE UND VERWENDUNG
DÉRIVÉS DE SULFONAMIDES AROMATIQUES INHIBANT PDI A3, LEUR SYNTHÈSE ET LEUR UTILISATION

(43) Date of publication of application: 16.11.2022
(73) Proprietor: UNIWERSYTET JAGIELLONSKI, 31-007 Kraków (PL)
(72) Inventor: CHLOPICKI, Stefan, 31-007 Kraków (PL); KALVINS, Ivars, 1006 Riga (LV); PRZYBOROWSKI, Kamil, 31-007 Kraków (PL); STOJAK, Marta, 31-007 Kraków (PL); ANDRIANOV, Victor, 1006 Riga (LV); DOMRACEVA, Ilona, 1006 Riga (LV); KANEPE-LAPSA, Iveta, 1006 Riga (LV); ZELENCOVA, Diana, 1006 Riga (LV); WIETRZYK, Joanna, 53-114 Wroclaw (PL); TURLEJ, Eliza, 53-114 Wroclaw (PL); STACHOWICZ, Martyna, 53-114 Wroclaw (PL); JAROSZ, Joanna, 53-114 Wroclaw (PL); MILCZAREK, Magdalena, 53-114 Wroclaw (PL); KRAMKOWSKI, Karol, 15-089 Bialystok (PL)
(74) Representative: Czarnik, Maciej
(86) International application number: PCT/PL2020/050005
(87) International publication number: WO 2021/141507

(56) References cited:
- WO-A1-2016/118639
- US-A- 4 267 174
- US-A1- 2008 242 677
- BALDWIN J E ET AL: "AMINO ACID SYNTHESIS VIA RING OPENING OF N-SULPHONYL AYIRIDINE-2- CARBOXYLATE ESTERS WITH ORGANOMETALLIC REAGENTS", TETRAHEDRON, ELSEVIER SCIENCE PUBLISHERS, AMSTERDAM, NL, vol. 49, no. 28, 1 January 1993 (1993-01-01), pages 6309 - 6330, XP002070483, ISSN: 0040-4020, DOI: 10.1016/S0040-4020(01)87968-0
- SZE KUI ET AL: "Electronic Supplementary Information Intermolecular (4+3) Cycloadditions of Aziridinyl Enolsilanes", 16 December 2013 (2013-12-16), pages S1 - S54, XP055719726, Retrieved from the Internet <URL:http://www.rsc.org/suppdata/cc/c3/c3cc48266a/c3cc48266a.pdf> [retrieved on 20200803]
- YOUHEI TAKEDA ET AL: "Supporting Information Asymmetric Synthesis of b 2 -Aryl Amino Acids through Pd- Catalyzed Enantiospecific and Regioselective Ring-Opening Suzuki-Miyaura Arylation of Aziridine-2-carboxylates", 3 June 2019 (2019-06-03), pages S1 - S126, XP055719731, Retrieved from the Internet <URL:https://chemistry-europe.onlinelibrary.wiley.com/action/downloadSupplement?doi=10.1002/chem.201902009&file=chem201902009-sup-0001-misc_information.pdf> [retrieved on 20200803]

## Description

### Field of the invention

The invention relates to a new group of aromatic sulphonamides derivatives and their synthesis and use for modulation of the activity of protein disulfide isomerase (PDI). More particularly, the invention provides small molecule inhibitors of PDI A3 that display antiplatelet, antithrombotic and anticancer activities.

### Background of the invention

Protein disulfide isomerase (PDI) is a thiol-oxidoreductase chaperone protein that is responsible for the isomerization, reduction, and oxidation of disulfide bonds. There are known over 20 members of the PDI family of enzymes. Structurally, prototypic PDI consists of four domains with a thioredoxin fold: a, b, b' and a', an extended C-terminus with KDEL ER retention sequence, and an interdomain linker x between the b' and a' domains. The a and a' domains are catalytically active, contain redox active CGHC active site and independently can perform oxidation and reduction reactions (Darby and Creighton, 1995). The b and b' domains are noncatalytic, but provide a substrate-binding domain of PDI. All four domains are needed to achieve the isomerization and chaperone activity of PDI. Besides its catalytic role involving thiols and disulfides, PDI also serves an essential structural role as the beta subunit of prolyl-4-hydroxylase (Koivu et al., 1987) and as a microsomal triglyceride transfer protein (Wetterau et al., 1990).

Protein disulfide isomerase (PDI) catalyze posttranslational disulfide bond formation and exchange and serve as chaperones during protein folding (Hatahet et al., 2009). PDI has been also identified at many diverse subcellular locations outside the endoplasmic reticulum. It has biological functions on the cell surfaces of lymphocytes, hepatocytes, platelets, and endothelial cells (Manickam et al., 2008; Hotchkiss et al., 1998; Essex et al., 1999; Burgess et al., 2000; Bennett et al., 2000; Hotchkiss et al., 1998; Burgess et al., 2000). PDI is rapidly secreted from both endothelial cells and platelets during thrombus formation *in vivo* (Cho et al., 2008; Jasuja et al., 2010). Inhibition of PDI using neutralizing antibodies blocks thrombus formation in several thrombosis models (Bennett et al., 2000; Cho et al., 2008; Jasuja et al., 2010; Reinhardt et al. 2008). Inhibition of PDI in these models abrogates not only platelet accumulation at the injury site but also fibrin generation. Although number of studies focused on PDIA1, this isoform is not the sole isoform of PDI that support thrombosis *in vivo* and platelet aggregation *in vitro.* In particular, PDIA3 has an important role in the activation of the αIIbβ3 fibrinogen receptor and platelet aggregation that cannot be replaced by PDIA1 (Essex DW and Wu Yi 2018). Thus, deficiency of PDI A3 results in blunted platelet aggregation (Essex DW and Wu Yi 2018) due to the impaired activation of GpIIb/IIIa receptor that is a major platelet receptor for fibrinogen binding and is required for platelet aggregation and thrombus formation (Essex DW and Wu Yi 2018).

Importantly, PDI has been implicated in proliferation, survival and metastasis of several types of cancers (Lee et al, 2017, Xu et al., 2012; Hashida et al., 2011; Lovat et al., 2008) and the important role of PDIA3 was postulated (Yang Z et al., 2018; Hettinghouse A et al., 2018).

These observations demonstrate a critical role for PDIA3 in various pathologies (Cho et al., 2008) including thrombus formation and development of cancer. In particular, PDI A3 is a novel interesting target to develop antiplatelet, antithrombotic effects and anticancer therapeutics
Several patent documents provide compounds that inhibit enzyme activity of cell-associated protein disulfide isomerase e.g. US20160145209A1, WO2016118639, US20150133514A1, US20020115713A1, WO2017011890A1, but none of them relates to aromatic sulphonamides derivatives.

Several substituted sulfonylaziridine-2-carboxylates can be obtained via tosylation of the nitrogen of commercially available aminoacid methyl ester hydrochlorides, followed by Mitsunobu ring closure, (Takeda et al. 2019).

Presently, majority of available inhibitors of PDI are sulfhydryl-reactive compounds that bind covalently and are non-selective, acting broadly on thiol isomerases (Karala et al., 2010) or are cytotoxic (Lovat et al., 2008; Khan et al., 2011). Thus, there is a clear need for new agents that interfere with PDI A3 activity but are otherwise selective and well tolerated in therapeutic contexts. Now it has been found that some of among N,N-disubstituted aromatic sulphonamides possess unique pharmacological properties associated with their ability to inhibit PDIA3 activity, which property affords their antiplatelet, antithrombotic, and anticancer activities.

### Detailed description of the invention

The invention relates to N,N-disubstituted aromatic sulphonamides in form of racemates or enantiomers, or a pharmaceutically acceptable salt, wherein the compounds are chosen from the list:
1-[[5-(Dimethylamino)-1-naphthyl]sulfony1]-N,N-dimethylaziridine-2-carboxamide (C-3371);
N,N-Dimethyl-1-(2-naphthylsulfony1)aziridine-2-carboxamide (C-3373);
Methyl 1-[[6-(dimethylamino)-1-naphthyl]sulfonyl]aziridine-2-caiboxylate (C-3375);
Methyl 1-[[6-(dimethylamino)-5-formyl-1-naphthyl]sulfonyl]aziridine-2-carboxylate (C-3376);
1-[[6-(Dimethylamino)-1-naphthyl]sulfony1]-N,N-dimethylaziridine-2-carboxamide (C-3377);
Methyl 1-[[6-(dimethylamino)-5-formyl-2-naphthyl]sulfonyl]aziridine-2-carboxylate (C-3390);
Methyl 1-[[4-(dimethylamino)-1-naphthyl]sulfonyl]aziridine-2-caiboxylate (C-3383);
1-[[4-(Dimethylamino)-1-naphthyl]sulfonyl]-N,N-dimethylaziridine-2-carboxamide (C-3385);
1-[[6-(Dimethylamino)-5-formyl-2-naphthy1]sulfony1]-N,N-dimethylaziridine-2-carboxamide (C-3391);
Methyl 1-[[6-(dimethylamino)-2-naphthyl]sulfonyl]aziridine-2-caiboxylate (C-3397);
1-[[6-(Dimethylamino)-2-naphthyl]sulfonyl]-N,N-dimethylaziridine-2-carboxamide (C-3398);
Methyl 1-[[5-(dimethylamino)-2-naphthyl]sulfonyl]aziridine-2-caiboxylate (C-3399);
1-[[5-(Dimethylamino)-2-naphthyl]sulfonyl]-N,N-dimethylaziridine-2-carboxamide (C-3400);
Methyl 1-[[8-(dimethylamino)-2-naphthyl]sulfonyl]aziridine-2-carboxylate (C-3427);
[1-(4-hexylpheny1)sulfonylaziridin-2-y1]-(1-piperidyl)methanone (C-3520)

The invention also relates to N,N-disubstituted aromatic sulphonamides or a pharmaceutically acceptable salt selected from the list for use in the treatment or prevention of diseases linked to PDI A3:
1-[[5-(Dimethylamino)-1-naphthyl]sulfonyl]-N,N-dimethylaziridine-2-carboxamide (C-3371);
N,N-Dimethyl-1-(2-naphthylsulfonyl)aziridine-2-carboxamide (C-3373);
Methyl 1-[[6-(dimethylamino)-1-naphthyl]sulfonyl]aziridine-2-caiboxylate (C-3375);
Methyl 1-[[6-(dimethylamino)-5-formyl-1-naphthyl]sulfonyl]aziridine-2-carboxylate (C-3376);
1-[[6-(Dimethylamino)-1-naphthyl]sulfonyl]-N,N-dimethylaziridine-2-carboxamide (C-3377);
Methyl 1-[[4-(dimethylamino)-1-naphthyl]sulfonyl]aziridine-2-caiboxylate (C-3383);
1-[[4-(Dimethylamino)-1-naphthyl]sulfonyl]-N,N-dimethylaziridine-2-carboxamide (C-3385);
Methyl 1-[[6-(dimethylamino)-5-formyl-2-naphthyl]sulfonyl]aziridine-2-carboxylate (C-3390);
1-[[6-(Dimethylamino)-5-formyl-2-naphthyl]sulfonyl]-N,N-dimethylaziridine-2-caiboxamide (C-3391);
Methyl 1-[[6-(dimethylamino)-2-naphthyl]sulfonyl]aziridine-2-carboxylate (C-3397);
1-[[6-(Dimethylamino)-2-naphthyl]sulfonyl]-N,N-dimethylaziridine-2-carboxamide (C-3398);
Methyl 1-[[5-(dimethylamino)-2-naphthyl]sulfonyl]aziridine-2-caiboxylate (C-3399);
1-[[5-(Dimethylamino)-2-naphthyl]sulfonyl]-N,N-dimethylaziridine-2-carboxamide (C-3400);
Methyl 1-[[8-(dimethylamino)-2-naphthyl]sulfonyl]aziridine-2-caiboxylate (C-3427);
[1-(4-hexylphenyl)sulfonylaziridin-2-yl]-(1-piperidyl)methanone (C-3520);
Methyl (2S)-1-[[6-(dimethylamino)-1-naphthyl]sulfonyl]aziridine-2-carboxylate (C-3548)

The invention also relates to N,N-disubstituted aromatic sulphonamides mentioned above for use in the treatment or prevention of diseases linked to PDI A3, for use in treatment and prevention of excessive platelet activation and thrombosis, in particular any disease from the list:
disease or condition is thrombosis, thrombotic diseases, in particular the thrombotic disease is acute myocardial infarction, stable angina, unstable angina, acute occlusion following coronary angioplasty and/or stent placement, transient ischemic attacks, cerebrovascular disease, peripheral vascular disease, placental insufficiency, atrial fibrillation, deep vein thrombosis or pulmonary embolism and other pathologies linked with excessive activation of platelets

The invention also relates to N,N-disubstituted aromatic sulphonamides mentioned above for use in the treatment or prevention of diseases linked to PDI A3, for use in treatment and prevention of cancer in particular any disease from the list:
gastrointestinal cancer, colorectal cancer, colon cancer, liver cancer, hepatocellular carcinoma, pancreatic cancer, biliary tract cancer, stomach cancer, genitourinary cancer, bladder cancer, testicular cancer, cervical cancer, malignant mesothelioma, osteogenic sarcoma, esophageal cancer, laryngeal cancer, prostate cancer, hormone-refractory prostate cancer, lung cancer, small cell lung cancer, non-small cell lung cancer, breast cancer, triple-negative breast cancer, breast cancer having a BRCA1 and/or BRCA2 gene mutation, hematological cancer, leukemia, acute lymphoblastic leukemia, acute myeloid leukemia, chronic lymphocytic leukemia, chronic myeloid leukemia, lymphoma, Hodgkin lymphoma, non-Hodgkin lymphoma, follicular lymphoma, diffuse large B-cell lymphoma, ovarian cancer, brain cancer, neuroblastoma, Ewing's sarcoma, kidney cancer, epidermoid cancer, skin cancer, melanoma, head and/or neck cancer, head and neck squamous cell carcinoma, and mouth cancer
The invention is set out in the appended set of claims. Embodiments which are not covered by the claims can reasonably be useful for highlighting specific aspects of the subject-matter.

The invention has been described in embodiments and figures non-limiting of the scope of protection, where:
- Fig. 1: shows inhibition of aggregation of human platelets by reference PDI inhibitors;
- Fig. 2, 3: show inhibition of aggregation of human platelets by PDI A3 inhibitors of the invention;
- Fig. 4: shows correlation between the inhibition of the PDI A3 and the inhibition of platelet aggregation for PDI A3 inhibitors of the invention;
- Fig. 5: shows antiplatelet antithrombotic effects in in vivo model of thrombosis for selected PDI-inhibitor and reference compounds; Fig. 5a i 5b;
- Fig. 6: shows effect of C-3399 (A) and C-3399 (B) on MDA-MB-231 cell transendothelial migration across endothelial monolayer;

### Example 1. Chemical synthesis

It is described below the general method for the preparation of N,N-disubstituted aromatic sulphonamides.

Aromatic or heteroaromatic sulphonic acid chloride (1 mmol) was added with stirring to the solution of the appropriated aziridine (1.1 mmol) and K₂CO₃ (2 mmol) in the mixture of 1 ml CHCl₃ + 1 ml water. The mixture was stirred for 24 h. at room temperature. Product was extracted with CHCl₃, and the solution dried over MgSO₄. The solvent was evaporated. The product was purified by chromatography (silica gel, petroleum ether/ethyl acetate 4:1=>1:2) to give corresponding aziridine aromatic N-sulfonamide. 1-(p-Tolylsulfonyl)aziridine-2-carbonitrile (C-3314) was prepared as described by Nadir, U. K. and Singh, A. Synthetic Communications, 34(7), 1337-1347; 2004. 1-(p-Tolylsulfonyl)aziridine-2-carbaldehyde (C-3262) was prepared as described by Lapinsky, D. J. and Bergmeier, S. C. Tetrahedron Letters, 42(49), 8583-8586; 2001. 1-(4-Butylphenyl)sulfonylaziridine-2-carbaldehyde (C-3273) was prepared using the same method. 1-[1-(p-Tolylsulfonyl)aziridin-2-y1]ethanone (C-3263) was prepared as described by Smith, A. B., and Kim, D.-S. Journal of Organic Chemistry, 71(7), 2547-2557; 2006. 1-[1-(4-Butylphenyl)sulfonylaziridin-2-yl]ethanone (C-3272) was prepared using the same method. Methyl (2S)-1-(p-tolylsulfonyl)aziridine-2-carboxylate (C-3535) was prepared as described by Qian, G.; Bai, M.; Gao, S.; Chen, H.; Zhou, S.; Cheng, H-G.; Yan, W.; Zhou, Q. Angewandte Chemie, International Edition (2018), 57(34), 10980-10984. Methyl (2R)-1-(p-tolylsulfonyl)aziridine-2-carboxylate (C-3539) was prepared as described by Smith, A. B. and Kim, D-S. Journal of Organic Chemistry (2006), 71(7), 2547-2557. Methyl (2S)-1-[[6-(dimethylamino)-1-naphthyl]sulfonyl]aziridine-2-caiboxylate (C-3548) and methyl (2R)-1-[[6-(dimethylamino)-1-naphthyl]sulfonyl]aziridine-2-carboxylate (C-3570) were prepared using methodology as described by Smith, A. B. and Kim, D-S. Journal of Organic Chemistry (2006), 71(7), 2547-2557.

Lithium 1-tosylaziridine-2-carboxylate (C-3612) was prepared as described by Baldwin, J. E.; Spivey, A. C.; Schofield, C. J.; Sweeney, J. B. Tetrahedron, 49(28), 6309-30; 1993

### Synthesis of 6-(dimethylamino)-5-formylnaphthalene-1-sulfonyl chloride.

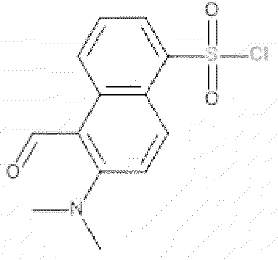

Into a 50 mL round-bottom flask, was placed 6-(dimethylamino)naphthalene-1-sulfonic acid (1.0 g, 3.98 mmol). To this was added CH₂Cl₂ (20 mL). To the mixture was added DMF (0.4 mL). To the above was added dropwise oxalyl dichloride (2.0 g, 15.74 mmol). The resulting solution was allowed to react with stirring for 24 h at room temperature. The reaction mixture was then quenched by the adding 50 mL of ice/salt. The resulting solution was extracted twice with 10 mL of CH₂Cl₂ and the organic layers combined and dried over Na₂SO₄. Solvent was evaporated under vacuum. The resulted 6-(dimethylamino)-5-formylnaphthalene-1-sulfonyl chloride (0.83 g 70%) was used on the next stage without additional purification. ¹H-NMR spectrum (400 MHz): (CDCl₃, HMDSO) δ: 10.30 (s, 1H), 9.55 (dt, *J*=8.7, 1.0 Hz, 1H), 8.87 (dd, *J*=9.7, 0.9 Hz, 1H), 8.18 (dd, *J*=7.6, 1.1 Hz, 1H), 7.66 (dd, *J*=8.7, 7.6 Hz, 1H), 7.60 (d, *J*=9.7 Hz, 1H), 3.22 (s, 6H).

The same method was used for the preparation of 6-(dimethylamino)-5-formylnaphthalene-2-sulfonyl chloride.

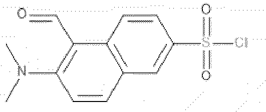

¹H-NMR spectrum (400 MHz): (CDCl₃, HMDSO) δ: 10.26 (s, 1H), 8.91 (d, *J*=9.1 Hz, 1H), 8.11 (d, *J*=9.1 Hz, 1H), 8.11 (m, 1H), 7.75 (dd, *J*=9.1, 2.0 Hz, 1H), 7.51 (d, *J*=9.1 Hz, 1H), 7.41 (d, *J*=9.1 Hz, 1H), 3.13 (s, 6H).

### Synthesis of 5-chloro-6-(methylamino)naphthalene-2-sulfonyl chloride.

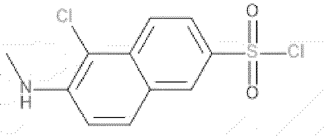

To a suspension of 6-(dimethylamino)naphthalene-2-sulfonic acid (1.0 g, 3.98 mmol). in POCl₃ (5 mL) was slowly added PCl₅ (3.7 g, 17.8 mmol). The resulting mixture was heated at 50 °C for 5 h before it was allowed to cool to room temperature and poured onto crushed ice. The aqueous mixture was stirred vigorously at 0 °C for 40 min. Product was extracted twice with 40 mL of CH₂Cl₂ and the organic layers combined and dried over Na₂SO₄. Solvent was evaporated under vacuum. The resulted 5-chloro-6-(methylamino)naphthalene-2-sulfonyl chloride was purified by chromatography (silica gel, petroleum ether/ethyl acetate 4:1). Yield 0.35 g (30%). ¹H-NMR spectrum (400 MHz): (CDCl₃, HMDSO) δ: 8.24 (d, *J=2.0* Hz, 1H), 8.15 (dt, *J*=9.2, 0.6 Hz, 1H), 7.95 (dd, *J*=9.2, 2.0 Hz, 1H), 7.86 (d, *J*=9.0 Hz, 1H), 7.22 (d, *J*=9.0 Hz, 1H), 3.11 (s, 3H).

**TABLE 1**

| | |
|---|---|
| C-3161 | Methyl 1-(p-tolylsulfonyl)aziridine-2-carboxylate |
| | Molecular formula: C₁₁H₁₃NO₄S; Molecular weight: 255.29; Melting point: 56-58°C; ¹H-NMR spectrum (400 MHz): (CDCl₃, HMDSO) δ: 7.84 (d, J=8.3 Hz, 2H), 7.35 (d, J=8.3 Hz, 2H), 3.74 (s, 3H), 3.34 (dd, J=7.1, 4.1 Hz, 1H), 2.76 (d, J=7.1 Hz, 1H), 2.56 (d, J=4.1 Hz, 1H), 2.45 (s, 3H); ¹³C-NMR spectrum (100 MHz): (CDCl₃, HMDSO) δ: 167.2, 145.2, 134.0, 129.9, 128.2, 52.9, 35.7, 32.0, 21.7; LCMS ESI⁻ (m/z): 254.2 [M-H]⁻, LCMS purity 100.00% |
| C-3212 | Methyl 1-(4-nitrophenyl)sulfonylaziridine-2-carboxylate |
| | Molecular formula: C₁₀H₁₀N₂O₆S; Molecular weight: 286.26; Melting point: 106-108°C; ¹H-NMR spectrum (400 MHz): (CDCl₃, HMDSO) δ: 8.41 (d, *J*=9.0 Hz, 2H), 8.18 (d, *J*=9.0 Hz, 2H), 3.76 (s, 3H), 3.46 (dd, *J*=7.1, 4.3 Hz, 1H), 2.89 (d, *J*=7.1 Hz, 1H), 2.66 (d, *J*=4.3 Hz, 1H); ¹³C-NMR spectrum (100 MHz): (CDCl₃, HMDSO) δ: 166.6, 150.9, 142.9, 129.5, 124.5, 53.1, 35.2, 32.5; LCMS ESI⁻ (m/z): 285.1 [M-H]⁻, LCMS purity 100.00% |
| C-3216 | 1-(4-Nitrophenyl)sulfonylaziridine-2-carboxamide |
| | Molecular formula: C₉H₉N₃O₅S; Molecular weight: 271.25; Melting point: 179-181°C; ¹H-NMR spectrum (400 MHz): (DMSO-d6, HMDSO) δ: 8.47 (d, J=8.9 Hz, 2H), 8.24 (d, J=8.8 Hz, 2H), 7.87 (br s, 1H), 7.47 (br s, 1H), 3.39 (dd, J=7.2, 4.5 Hz, 1H), 2.79 (d, J=7.2 Hz, 1H), 2.58 (d, J=4.5 Hz, 1H); ¹³C-NMR spectrum (100 MHz): (DMSO-d6, HMDSO) δ: 166.5, 151.2, 142.6, 130.0, 125.3, 37.6, 32.2; LCMS ESI⁺ (m/z): 271.9 [M+H]⁺; Impurities: 3.67% (210 nm); 2.24% (254 nm) |
| C-3218 | 1-(4-Aminophenyl)sulfonylaziridine-2-carboxamide |
| | Molecular formula: C₉H₁₁N₃O₃S; Molecular weight: 241.27; Melting point: 139-141°C; ¹H-NMR spectrum (400 MHz): (CDCl₃, HMDSO) δ: 7.72 (s, 1H), 7.50 (d, *J*=8.7 Hz, 2H), 7.34 (s, 1H), 6.65 (d, *J*=8.7 Hz, 2H), 6.25 (s, 2H), 3.01 (dd, *J*=7.1, 4.1 Hz, 1H), 2.41 (d, *J*=7.1 Hz, 1H), 2.35 (d, *J*=4.1 Hz, 1H); ¹³C-NMR spectrum (100 MHz): (DMSO-d₆, HMDSO) δ: 167.5, 154.6, 130.5, 120.5, 113.2, 36.8, 31.2; LCMS ESI⁻ (m/z): 242.2 [M-H]⁻, LCMS purity 100.00% |
| C-3220 | 1-(p-Tolylsulfonyl)aziridine-2-carboxamide |
| | Molecular formula: C₁₀H₁₂N₂O₃S; Molecular weight: 240.28; Melting point: 118-120°C; ¹H-NMR spectrum (400 MHz): (CDCl₃, HMDSO) δ: 7.82 (d, *J*=8.2 Hz, 2H), 7.37 (d, *J*=8.2 Hz, 2H), 6.11 (s, 1H), 5.76 (s, 1H), 3.23 (dd, *J*=7.7, 4.2 Hz, 1H), 2.80 (d, *J*=7.7 Hz, 1H), 2.48 (s, 3H), 2.43 (d, *J*=4.2 Hz, 1H); ¹³C-NMR spectrum (100 MHz): (CDCl₃, HMDSO) δ: 168.3, 145.6, 133.4, 130.0, 128.2, 37.6, 33.1, 21.7; LCMS ESI⁻ (m/z): 241.2 [M-H]⁻, LCMS purity 100.00% |
| C-3251 | Methyl 1-(benzenesulfonyl)aziridine-2-carboxylate |
| | Molecular formula: C₁₀H₁₁NO₄S; Molecular weight: 241.26; Melting point: oil; ¹H-NMR spectrum (400 MHz): (CDCl₃) δ: 7.00-8.94 (m, 2H), 7.71-7.64 (m, 1H), 7.60-7.53 (m, 2H), 3.74 (s, 3H), 3.37 (dd, J=7.1, 4.1 Hz, 1H), 2.79 (d, J=7.1 Hz, 1H), 2.58 (d, J=4.1 Hz, 1H); ¹³C-NMR spectrum (100 MHz): (CDCl₃) δ: 167.3, 137.2, 134.3, 129.4, 128.3, 53.1, 35.9, 32.3; LCMS ESI⁺ (m/z): 242.1 [M+H]⁺, LCMS purity 100.00% |
| C-3256 | Methyl 1-[4-(trifluoromethyl)phenyl]sulfonylaziridine-2-carboxylate |
| | Molecular formula: C₁₁H₁₀F₃NO₄S; Molecular weight: 309.26; Melting point: 68-70°C; ¹H-NMR spectrum (400 MHz): (CDCl₃, HMDSO) δ: 8.12 (d, *J*=8.4 Hz, 2H), 7.84 (d, *J*=8.4 Hz, 2H), 3.76 (s, 3H), 3.43 (dd, *J=7.1,* 4.2 Hz, 1H), 2.85 (dd, *J*=7.1, 0.9 Hz, 1H), 2.63 (dd, *J*=4.2, 0.9 Hz, 1H); ¹³C-NMR spectrum (100 MHz): (CDCl₃, HMDSO) δ: 166.8, 140.8, 135.7 (q, *J*=33.1 Hz), 128.7, 126.4 (q, *J*=3.7 Hz), 123.0 (q, *J*=273.4 Hz), 53.0, 36.0, 32.3; LCMS ESI⁻ (m/z): 308.2 [M-H]⁻, LCMS purity 100.00% |
| C-3257 | Methyl 1-(4-butylphenyl)sulfonylaziridine-2-carboxylate |
| | Molecular formula: C₁₄H₁₉NO₄S; Molecular weight: 297.37; Melting point: oil; ¹H-NMR spectrum (400 MHz): (CDCl₃, HMDSO) δ: 7.86 (d, J=8.4 Hz, 2H), 7.36 (d, J=8.4 Hz, 2H), 3.74 (s, 3H), 3.35 (dd, J=7.1, 4.1 Hz, 1H), 2.76 (d, J=7.1 Hz, 1H), 2.70 (t, J=7.7 Hz, 2H), 2.56 (d, J=4.1 Hz, 1H), 1.58-1.67 (m, 2H), 1.36 (sextet, J=7.5 Hz, 2H), 0.94 (d, J=7.4 Hz, 3H); ¹³C-NMR spectrum (100 MHz): (CDCl₃, HMDSO) δ: 167.3, 150.1, 134.1, 129.2, 128.2, 52.9, 35.6, 35.6, 32.1, 32.1, 22.2, 13.8; LCMS ESI⁺ (m/z): 298.2 [M+H]⁺, LCMS purity 100% |
| C-3262 | 1-(p-Tolylsulfonyl)aziridine-2-carbaldehyde |
| | Molecular formula: C₁₀H₁₁NO₄S; Molecular weight: 225.26; Melting point: oil; ¹H-NMR spectrum (400 MHz): (CDCl₃, HMDSO) δ: 8.95 (d, *J*=5.9 Hz, 1H), 7.84 (d, *J*=8.3 Hz, 2H), 7.38 (d, *J*=8.3 Hz, 2H), 3.27 (ddd, *J*=7.4, 5.9, 4.0 Hz, 1H), 2.93 (d, *J*=7.4 Hz, 1H), 2.58 (d, *J*=4.0 Hz, 1H), 2.47 (s, 3H); ¹³C-NMR spectrum (100 MHz): (CDCl₃, HMDSO) δ: 194.3, 145.6, 133.7, 130.0, 128.2, 41.9, 30.1, 21.7; LCMS ESI⁺ (m/z): 226.2 [M+H]⁺, LCMS purity 94.70% |
| C-3263 | 1-[1-(p-Tolylsulfonyl)aziridin-2-y1]ethanone |
| | Molecular formula: C₁₁H₁₃NO₃S; Molecular weight: 239.29; Melting point: 90-92°C; ¹H-NMR spectrum (400 MHz): (CDCl₃, HMDSO) δ: 7.84 (d, J=8.3 Hz, 2H), 7.37 (d, J=8.3 Hz, 2H), 3.29 (dd, J=7.4, 4.1 Hz, 1H), 2.80 (d, J=7.1 Hz, 1H), 2.49 (d, J=4.1 Hz, 1H), 2.46 (s, 3H), 2.07 (s, 3H); ¹³C-NMR spectrum (100 MHz): (CDCl₃, HMDSO) δ: 201.4, 145.3, 133.9, 129.9, 128.1, 41.9, 31.8, 25.9, 21.7; LCMS ESI⁺ (m/z): 240.2 [M+H]+ , LCMS purity 100%; Elemental analysis: Found [%]: C 55.07, H 5.35, N 5.74; Calculated [%]: C 55.21, H 5.48, N 5.85 |
| C-3270 | Methyl 1-(4-dodecylphenyl)sulfonylaziridine-2-carboxylate |
| | Molecular formula: C₂₂H₃₅NO₄S; Molecular weight: 409.58; Melting point: oil; ¹H-NMR spectrum (400 MHz): (CDCl₃, HMDSO) δ: 7.86 (d, *J*=8.4 Hz, 2H), 7.35 (d, *J*=8.4 Hz, 2H), 3.74 (s, 3H), 3.36 (dd, *J*=7.1, 4.1 Hz, 1H), 2.77 (d, *J*=7.1 Hz, 1H), 2.69 (t, *J*=7.7 Hz, 2H), 2.56 (d, *J*=4.1 Hz, 1H), 1.63 (quintet, *J*=7.5 Hz, 2H), 1.20-1.38 (m, 18H), 0.87 (d, *J*=6.9 Hz, 3H); ¹³C-NMR spectrum (100 MHz): (CDCl₃, HMDSO) δ: 167.3, 150.2, 134.1, 129.2, 128.2, 52.9, 36.0, 35.6, 32.1, 31.9, 29.6, 29.5, 29.4, 29.3, 29.2, 22.7, 14.1; LCMS ESI⁺ (m/z): 410.4 [M+H]⁺, LCMS purity 100.00% |
| C-3271 | Methyl 1-(4-butoxyphenyl)sulfonylaziridine-2-carboxylate |
| | Molecular formula: C₁₄H₁₉NO₅S; Molecular weight: 313.37; Melting point: oil; ¹H-NMR spectrum (400 MHz): (CDCl₃, HMDSO) δ: 7.87 (d, J=8.4 Hz, 2H), 7.00 (d, J=8.4 Hz, 2H), 4.04 (t, J=6.5 Hz, 2H), 3.74 (s, 3H), 3.32 (dd, J=7.1, 4.1 Hz, 1H), 2.74 (d, J=7.1 Hz, 1H), 2.55 (d, J=4.1 Hz, 1H), 1.80 (quintet, J=6.7 Hz, 2H), 1.50 (sextet, J=7.5 Hz, 2H), 0.99 (t, J=7.4 Hz, 3H); ¹³C-NMR spectrum (100 MHz): (CDCl₃, HMDSO) δ: 167.3, 163.7, 130.4, 127.9, 114.9, 68.3, 52.8, 35.6, 32.0, 30.9, 19.1, 13.7; LCMS ESI⁺ (m/z): 314.3 [M+H]⁺; LCMS purity 93.03% |
| C-3272 | 1-[1-(4-Butylphenyl)sulfonylaziridin-2-yl]ethanone |
| | Molecular formula: C₁₄H₁₉NO₃S; Molecular weight: 281.37; Melting point: oil; ¹H-NMR spectrum (400 MHz): (CDCl₃, HMDSO) δ: 7.84 (d, J=8.4 Hz, 2H), 7.36 (d, J=8.3 Hz, 2H), 3.29 (dd, J=7.4, 4.1 Hz, 1H), 2.70 (t, J=7.7 Hz, 2H), 2.79 (d, J=7.4 Hz, 1H), 2.49 (d, J=4.1 Hz, 1H), 2.46 (s, 3H), 2.07 (s, 3H), 1.57-1.67 (m, 2H), 1.36 (sextet, J=7.5 Hz, 2H), 0.93 (t, J=7.4 Hz, 3H); ¹³C-NMR spectrum (100 MHz): (CDCl₃, HMDSO) δ: 201.5, 150.2, 134.0, 129.3, 128.2, 41.9, 35.6, 33.0, 31.9, 25.9, 22.2, 13.8; LCMS ESI⁺ (m/z): 282.3 [M+H]⁺ , LCMS purity 96.65% |
| C-3273 | 1-(4-Butylphenyl)sulfonylaziridine-2-carbaldehyde |
| | Molecular formula: C₁₃H₁₇NO₃S; Molecular weight: 267.34; Melting point: oil; ¹H-NMR spectrum (400 MHz): (CDCl₃, HMDSO) δ: 8.96 (d, *J*=5.8 Hz, 1H), 7.85 (d, *J*=8.4 Hz, 2H), 7.38 (d, *J*=8.3 Hz, 2H), 3.28 (ddd, *J*=7.4, 5.8, 4.0 Hz, 1H), 2.93 (d, *J*=7.4 Hz, 1H), 2.71 (t, *J*=7.8 Hz, 2H), 2.58 (d, *J*=4.0 Hz, 1H), 1.57-1.68 (m, 2H), 1.37 (sextet, *J*=7.5 Hz, 2H), 0.94 (t, *J*=7.5 Hz, 3H); ¹³C-NMR spectrum (100 MHz): (CDCl₃, HMDSO) δ: 194.3, 150.4, 133.8, 129.4, 128.2, 41.8, 35.6, 33.0, 30.1, 22.2, 13.8; LCMS ESI⁺ (m/z): 268.4 [M+H]⁺, LCMS purity 100.00% |
| C-3281 | Methyl 1-(4-pentylphenyl)sulfonylaziridine-2-carboxylate |
| | Molecular formula: C₁₅H₂₁NO₄S; Molecular weight: 311.40; Melting point: oil; ¹H-NMR spectrum (400 MHz): (CDCl₃, HMDSO) δ: (CDCl₃, HMDSO) δ: 7.86 (d, *J*=8.4 Hz, 2H), 7.39 (d, *J*=8.4 Hz, 2H), 3.74 (s, 3H), 3.36 (dd, *J*=7.1, 4.1 Hz, 1H), 2.77 (d, *J*=7.1 Hz, 1H), 2.69 (t, *J*=7.8 Hz, 2H), 2.56 (d, *J*=4.1 Hz, 1H), 1.64 (quintet, *J*=7.5 Hz, 2H), 1.27-1.40 (m, 4H), 0.90 (t, *J*=7.0 Hz, 3H); ¹³C-NMR spectrum (100 MHz): (CDCl₃, HMDSO) δ: 167.2, 150.1, 134.1, 129.2, 128.2, 52.8, 35.8, 35.6, 32.1, 31.3, 30.6, 22.4, 13.9; LCMS ESI⁺ (m/z): 312.4 [M+H]⁺, LCMS purity 100.00% |
| C-3287 | Methyl 1-(4-hexylphenyl)sulfonylaziridine-2-carboxylate |
| | Molecular formula: C₁₆H₂₃NO₄S; Molecular weight: 325.42; Melting point: oil; ¹H-NMR spectrum (400 MHz): (CDCl₃) δ: 7.88-7.83 (m, 2H), 7.38-7.32 (m, 2H), 3.74 (s, 3H), 3.35 (dd, J=7.1, 4.1 Hz, 1H), 2.76 (d, J=7.1 Hz, 1H), 2.73-2.65 (m, 2H), 2.56 (d, J=4.1 Hz, 1H), 1.68-1.58 (m, 2H), 1.38-1.24 (m, 6H), 0.92-0.84 (m, 3H); ¹³C-NMR spectrum (100 MHz): (CDCl₃) δ: 167.4, 150.3, 134.3, 129.4, 128.4, 53.0, 36.1, 35.8, 32.2, 31.7, 31.1, 29.0, 22.7, 14.2; LCMS ESI⁺ (m/z): 326.2 [M+H]+ , LCMS purity 99.43% |
| C-3288 | Methyl 1-(4-heptylphenyl)sulfonylaziridine-2-carboxylate |
| | Molecular formula: C₁₇H₂₅NO₄S; Molecular weight: 339.45; Melting point: oil; ¹H-NMR spectrum (400 MHz): (CDCl₃, HMDSO) δ: 7.86 (d, *J*=8.5 Hz, 2H), 7.36 (d, *J*=8.5 Hz, 2H), 3.74 (s, 3H), 3.36 (dd, *J*=7.1, 4.1 Hz, 1H), 2.77 (d, *J*=7.1 Hz, 1H), 2.69 (t, *J*=7.7 Hz, 2H), 2.56 (d, *J*=4.1 Hz, 1H), 1.63 (quintet, *J*=7.5 Hz, 2H), 1.22-1.37 (m, 8H), 0.88 (t, *J*=7.1 Hz, 3H); ¹³C-NMR spectrum (100 MHz): (CDCl₃, HMDSO) δ: 167.3, 150.1, 134.1, 129.2, 128.2, 52.9, 35.9, 35.6, 32.1, 31.7, 31.0, 29.1, 29.0, 22.6, 14.0; LCMS ESI⁺ (m/z): 340.4 [M+H]⁺, LCMS purity 100.00% |
| C-3290 | Methyl 1-(4-tert-butylphenyl)sulfonylaziridine-2-carboxylate |
| | Molecular formula: C₁₄H₁₉NO₄S; Molecular weight: 297.37; Melting point: oil; ¹H-NMR spectrum (400 MHz): (CDCl₃, HMDSO) δ: 7.88 (d, *J*=8.6 Hz, 2H), 7.57 (d, *J*=8.6 Hz, 2H), 3.74 (s, 3H), 3.37 (dd, *J*=7.1, 4.1 Hz, 1H), 2.76 (d, *J*=7.1 Hz, 1H), 2.56 (d, *J*=4.1 Hz, 1H), 1.35 (s, 9H); ¹³C-NMR spectrum (100 MHz): (CDCl₃, HMDSO) δ: 167.3, 158.1, 133.9, 128.0, 126.3, 52.9, 35.6, 35.3, 32.1, 31.0; LCMS ESI⁺ (m/z): 398.3 [M+H]⁺, LCMS purity 100.00% |
| C-3291 | Methyl 1-(4-phenylphenyl)sulfonylaziridine-2-carboxylate |
| | Molecular formula: C₁₆H₁₅NO₄S; Molecular weight: 317.36; Melting point: 90-92°C; ¹H-NMR spectrum (400 MHz): (CDCl₃, HMDSO) δ: 8.03 (d, *J*=8.8 Hz, 2H), 7.77 (d, *J*=8.8 Hz, 2H), 7.60-7.62 (m, 2H), 7.41-7.52 (m, 3H), 3.76 (s, 3H), 3.41 (dd, *J*=7.1, 4.1 Hz, 1H), 2.82 (d, *J*=7.1 Hz, 1H), 2.61 (d, *J*=4.1 Hz, 1H); ¹³C-NMR spectrum (100 MHz): (CDCl₃, HMDSO) δ: 167.2, 147.1, 139.0, 135.5, 129.1, 128.7, 128.7, 127.9, 127.4, 52.9, 35.7, 32.1; LCMS ESI⁺ (m/z): 318.3 [M+H]⁺, LCMS purity 100.00% |
| C-3292 | Methyl 1-(2-naphthylsulfonyl)aziridine-2-carboxylate |
| | Molecular formula: C₁₄H₁₃NO₄S; Molecular weight: 291.32; Melting point: oil; ¹H-NMR spectrum (400 MHz): (CDCl₃, HMDSO) δ: 8.52-8.54 (m, 1H), 7.99-8.03 (m, 2H), 7.93-7.97 (m, 2H), 7.67-7.72 (m, 1H), 7.62-7.67 (m, 1H), 3.73 (s, 3H), 3.44 (dd, J=7.1, 4.1 Hz, 1H), 2.85 (d, J=7.1 Hz, 1H), 2.60 (d, J=4.1 Hz, 1H); ¹³C-NMR spectrum (100 MHz): (CDCl₃, HMDSO) δ: 167.1, 135.5, 133.9, 132.0, 129.9, 129.6, 129.5, 129.5, 128.0, 127.8, 122.8, 52.9, 35.8, 32.1; LCMS ESI⁺ (m/z): 292.3 [M+H]+ , LCMS purity 97.48% |
| C-3294 | Methyl 1-(4-methoxyphenyl)sulfonylaziridine-2-carboxylate |
| | Molecular formula: C₁₁H₁₃NO₅S; Molecular weight: 271.29; Melting point: 73-75 °C; ¹H-NMR spectrum (400 MHz): (CDCl₃, HMDSO) δ: 7.89 (d, J=9.1 Hz, 2H), 7.01 (d, J=9.1 Hz, 2H), 3.89 (s, 3H), 3.74 (s, 3H), 3.32 (dd, J=7.1, 4.1 Hz, 1H), 2.75 (d, J=7.1 Hz, 1H), 2.55 (d, J=4.1 Hz, 1H); ¹³C-NMR spectrum (100 MHz): (CDCl₃, HMDSO) δ: 167.3, 164.1, 130.4, 128.3, 114.5, 55.7, 52.8, 35.7, 32.0; LCMS ESI⁺ (m/z): 272.3 [M+H]+ , LCMS purity 100% |
| C-3295 | Methyl 1-(4-isopropylphenyl)sulfonylaziridine-2-carboxylate |
| | Molecular formula: C₁₃H₁₇NO₄S; Molecular weight: 283.34; Melting point: oil; ¹H-NMR spectrum (400 MHz): (CDCl₃, HMDSO) δ: 7.88 (d, J=8.6 Hz, 2H), 7.40 (d, J=8.6 Hz, 2H), 3.75 (s, 3H), 3.36 (dd, J=7.1, 4.1 Hz, 1H), 3.00 (septet, J=6.9 Hz, 1H), 2.76 (d, J=7.1 Hz, 1H), 2.56 (d, J=4.1 Hz, 1H), 1.28 (d, J=6.9 Hz, 6H); ¹³C-NMR spectrum (100 MHz): (CDCl₃, HMDSO) δ: 167.3, 155.9, 134.2, 128.3, 127.4, 52.8, 35.6, 34.2, 32.1, 23.5; LCMS ESI+ (m/z): 284.3 [M+H]+, LCMS purity 100% |
| C-3296 | Methyl 1-(4-chlorophenyl)sulfonylaziridine-2-carboxylate |
| | Molecular formula: C₁₀H₁₀ClNO₄S; Molecular weight: 275.71; Melting point: oil; ¹H-NMR spectrum (400 MHz): (CDCl₃, HMDSO) δ: 7.91 (d, *J*=8.7 Hz, 2H), 7.55 (d, *J*=8.7 Hz, 2H), 3.75 (s, 3H), 3.38 (dd, *J*=7.1, 4.1 Hz, 1H), 2.80 (d, *J*=7.1 Hz, 1H), 2.60 (d, *J*=4.1 Hz, 1H); ¹³C-NMR spectrum (100 MHz): (CDCl₃, HMDSO) δ: 166.9, 140.9, 135.6, 129.6, 129.6, 53.0, 35.9, 32.2; LCMS ESI⁺ (m/z): 276.2 [M+H]⁺, LCMS purity 100.00% |
| C-3297 | Methyl 1-(2-fluoro-4-methyl-phenyl)sulfonylaziridine-2-carboxylate |
| | Molecular formula: C₁₁H₁₂FNO₄S; Molecular weight: 273.26; Melting point: oil; ¹H-NMR spectrum (400 MHz): (CDCl₃, HMDSO) δ: 7.82 (dd, *J*=8.0, 7.3 Hz, 1H), 7.10-7.13 (m, 1H), 7.04-7.08 (m, 1H), 3.77 (s, 3H), 3.50 (ddd, *J*=7.1, 4.3, 0.9 Hz, 1H), 2.95 (dd, *J*=7.1, 1.2 Hz, 1H), 2.66 (d, *J*=4.3 Hz, 1H), 2.44 (s, 3H); ¹³C-NMR spectrum (100 MHz): (CDCl₃, HMDSO) δ: 167.2, 159.3 (d, *J*=258.3 Hz,), 148.8 (d, *J*=8.5 Hz,), 130.3, 125.2 (d, *J*=3.0 Hz,), 122.4 (d, *J*=14.5 Hz,), 117.8 (d, *J*=20.9 Hz,),, 52.9, 35.9, 32.4, 21.6; LCMS ESI⁺ (m/z): 274.3 [M+H]⁺, LCMS purity 96.13% |
| C-3299 | Methyl 1-(4-propylphenyl)sulfonylaziridine-2-carboxylate |
| | Molecular formula: C₁₃H₁₇NO₄S; Molecular weight: 283.34; Melting point: oil; ¹H-NMR spectrum (400 MHz): (CDCl₃, HMDSO) δ: 7.86 (d, *J*=8.5 Hz, 2H), 7.36 (d, *J*=8.5 Hz, 2H), 3.74 (s, 3H), 3.36 (dd, *J*=7.1, 4.1 Hz, 1H), 2.77 (d, *J*=7.1 Hz, 1H), 2.68 (t, *J*=7.6 Hz, 2H), 2.56 (d, *J*=4.1 Hz, 1H), 1.68 (sextet, *J*=7.5 Hz, 2H), 0.95 (t, *J*=7.4 Hz, 3H); ¹³C-NMR spectrum (100 MHz): (CDCl₃, HMDSO) δ: (CDCl₃, HMDSO) δ: 167.2, 149.8, 134.2, 129.3, 128.2, 52.9, 37.9, 35.6, 32.1, 24.1, 13.6; LCMS ESI⁺ (m/z): 284.3 [M+H]⁺, LCMS purity 100.00% |
| C-3303 | Methyl 1-(m-tolylsulfonyl)aziridine-2-carboxylate |
| | Molecular formula: C₁₁H₁₃NO₄S; Molecular weight: 255.29; Melting point: oil; ¹H-NMR spectrum (400 MHz): (CDCl₃, HMDSO) δ: 7.88-7.80 (m, 1H), 7.74-7.77 (m, 1H), 7.46-7.50 (m, 1H), 7.42-7.46 (m, 1H), 3.74 (s, 3H), 3.37 (dd, *J*=7.1, 4.1 Hz, 1H), 2.78 (d, *J*=7.1 Hz, 1H), 2.57 (d, *J*=4.1 Hz, 1H), 2.45 (s, 3H); ¹³C-NMR spectrum (100 MHz): (CDCl₃, HMDSO) δ: 167.2, 139.6, 136.8, 135.0, 129.1, 128.5, 125.2, 52.9, 35.6, 32.1, 24.3; LCMS ESI⁺ (m/z): 256.2 [M+H]⁺, LCMS purity 97.26% |
| C-3304 | Methyl 1-(4-propylphenyl)sulfonylaziridine-2-carboxylate |
| | Molecular formula: C₁₃H₁₇NO₄S; Molecular weight: 283.34; Melting point:106-108°C ¹H-NMR spectrum (400 MHz): (CDCl₃, HMDSO) δ: 6.98 (s, 2H), 3.74 (s, 3H), 3.34 (dd, J=7.1, 4.1 Hz, 1H), 2.79 (d, J=7.1 Hz, 1H), 2.69 (s, 6H), 2.51 (d, J=4.1 Hz, 1H), 2.32 (s, 3H); ¹³C-NMR spectrum (100 MHz): (CDCl₃, HMDSO) δ: 167.5, 143.6, 140.4, 131.9, 131.7, 52.7, 34.7, 31.7, 22.9, 21.0 |
| | LCMS ESI+ (m/z): 284.3 [M+H]+ , LCMS purity 100% |
| C-3305 | Methyl 1-[3-(trifluoromethyl)phenyl]sulfonylaziridine-2-carboxylate Molecular formula: C₁₁H₁₀F₃NO₄S; Molecular weight: 309.26; Melting point: oil; ¹H-NMR spectrum (400 MHz): (CDCl₃, HMDSO) δ: 8.23-8.26 (m, 1H), 8.16-8.20 (m, 1H), 7.92-7.96 (m, 1H), 7.71-7.77 (m, 1H), 3.76 (s, 3H), 3.45 (dd, *J*=7.1, 4.2 Hz, 1H), 2.86 (dd, *J*=7.1, 0.9 Hz, 1H), 2.64 (dd, *J*=4.2, 0.9 Hz, 1H); ¹³C-NMR spectrum (100 MHz): (CDCl₃, HMDSO) δ: 166.8, 138.5, 132.0 (q, J=34.1 Hz), 131.4, 130.8 (q, *J*=3.6 Hz), 130.1, 125.2 (q, *J*=3.8 Hz), 123.0 (q, *J*=273.8 Hz), 53.0, 36.0, 32.4; LCMS ESI⁻ (m/z): 310.2 [M+H]⁺, LCMS purity 100.00% |
| C-3308 | Methyl 1-(4-acetamidophenyl)sulfonylaziridine-2-carboxylate |
| | Molecular formula: C₁₂H₁₄N₂O₅S; Molecular weight: 298.31; Melting point: oil; ¹H-NMR spectrum (400 MHz): (CDCl₃, HMDSO) δ: (CDCl₃, HMDSO) δ: 7.73-7.95 (m, 3H), 7.67 (d, J=8.9 Hz, 2H), 3.74 (s, 3H), 3.43 (dd, *J*=7.1, 4.1 Hz, 1H), 2.76 (d, *J*=7.1 Hz, 1H), 2.56 (d, *J*=4.1 Hz, 1H), 2.21 (s, 3H); ¹³C-NMR spectrum (100 MHz): (CDCl₃, HMDSO) δ: 168.9, 167.4, 143.4, 131.0, 129.5, 119.3, 53.0, 35.7, 32.1, 24.7; LCMS ESI⁺ (m/z): 299.2 [M+H]⁺, LCMS purity 94.91% |
| C-3311 | Methyl 1-(1-naphthylsulfonyl)aziridine-2-carboxylate |
| | Molecular formula: C₁₄H₁₃NO₄S; Molecular weight: 291.32; Melting point: 102-104°C; ¹H-NMR spectrum (400 MHz): (CDCl₃, HMDSO) δ: 8.90 (ddd, J=8.6, 1.8, 0.8 Hz, 1H), 8.24 (dd, J=7.4, 1.3 Hz, 1H), 8.15 (dt, J=8.2, 1.2 Hz, 1H), 7.95 (ddd, J=8.2, 1.3, 0.6 Hz, 1H), 7.73 (ddd, J=8.2, 6.9, 1.3 Hz, 1H), 7.63 (ddd, J=8.2, 6.9, 1.2 Hz, 1H), 7.57 (dd, J=8.2, 7.4 Hz, 1H), 3.70 (s, 3H), 3.49 (dd, J=7.1, 4.1 Hz, 1H), 2.89 (d, J=7.1 Hz, 1H), 2.59 (d, J=4.1 Hz, 1H); ¹³C-NMR spectrum (100 MHz): (CDCl₃, HMDSO) δ: 167.1, 135.7, 134.2, 132.6, 130.0, 129.0, 128.7, 128.6, 127.2, 125.5, 124.0, 52.8, 35.9, 32.5; LCMS ESI+ (m/z): 292.3 [M+H]+ , LCMS purity 98.48% |
| C-3314 | 1-(p-Tolylsulfonyl)aziridine-2-carbonitrile |
| | Molecular formula: C₁₀H₁₀N₂O₂S; Molecular weight: 222.26; Melting point: 83-85°C; ¹H-NMR spectrum (400 MHz): (CDCl₃, HMDSO) δ: 7.85 (d, J=8.4 Hz, 2H), 7.41 (d, J=8.4 Hz, 2H), 3.22 (dd, J=7.0, 4.0 Hz, 1H), 2.89 (d, J=7.0 Hz, 1H), 2.65 (d, J=4.0 Hz, 1H), 2.49 (s, 3H); ¹³C-NMR spectrum (100 MHz): (CDCl₃, HMDSO) δ: 146.1, 133.1, 130.2, 128.2, 115.0, 32.1, 23.4, 21.7; LCMS ESI- (m/z): 221.2 [M-H]-, LCMS purity 100.00% |
| C-3316 | Methyl 1-(4-cyclohexylphenyl)sulfonylaziridine-2-carboxylate |
| | Molecular formula: C₁₆H₂₁NO₄S; Molecular weight: 323.12; Melting point: oil; ¹H-NMR spectrum (400 MHz): (CDCl₃, HMDSO) δ: 7.87 (d, *J*=8.5 Hz, 2H), 7.38 (d, *J*=8.5 Hz, 2H), 3.74 (s, 3H), 3.36 (dd, *J*=7.1, 4.1 Hz, 1H), 2.76 (d, *J*=7.1 Hz, 1H), 2.56-2.65 (m, 1H), 2.56 (d, *J*=4.1 Hz, 1H), 1.82-1.94 (m, 4H), 1.73-1.82 (m, 1H), 1.34-1.50 (m, 4H), 1.20-1.33 (m, 1H); ¹³C-NMR spectrum (100 MHz): (CDCl₃, HMDSO) δ: 167.3, 155.0, 134.1, 128.3, 127.7, 52.8, 44.6, 35.5, 34.0, 32.1, 26.6, 25.9; LCMS ESI⁺ (m/z): 324.3 [M+H]⁺, LCMS purity 98.89% |
| C-3319 | Methyl 1-(4-octylphenyl)sulfonylaziridine-2-carboxylate |
| | Molecular formula: C₁₈H₂₇NO₄S; Molecular weight: 353.48; Melting point: oil; ¹H-NMR spectrum (400 MHz): (CDCl₃, HMDSO) δ: 7.86 (d, *J*=8.5 Hz, 2H), 7.36 (d, *J*=8.5 Hz, 2H), 3.74 (s, 3H), 3.36 (dd, *J*=7.1, 4.1 Hz, 1H), 2.77 (d, *J*=7.1 Hz, 1H), 2.69 (t, *J*=7.7 Hz, 2H), 2.56 (d, *J*=4.1 Hz, 1H), 1.63 (quintet, *J*=7.6 Hz, 2H), 1.22-1.36 (m, 10H), 0.88 (t, *J*=7.1 Hz, 3H); ¹³C-NMR spectrum (100 MHz): (CDCl₃, HMDSO) δ: 167.3, 150.1, 134.1, 129.2, 128.2, 52.8, 35.9, 35.6, 32.1, 31.8, 31.0, 29.3, 29.1, 29.0, 22.6, 14.0; LCMS ESI⁺ (m/z): 354.4 [M+H]⁺, LCMS purity 100.00% |
| C-3320 | Methyl 1-(o-tolylsulfonyl)aziridine-2-carboxylate |
| | Molecular formula: C₁₁H₁₃NO₄S; Molecular weight: 255.29; Melting point: oil; ¹H-NMR spectrum (400 MHz): (CDCl₃, HMDSO) δ: 7.94 (dd, *J*=7.8, 1.4 Hz, 1H), 7.53 (dt, *J*=7.5, 1.4 Hz, 1H), 7.32-7.39 (m, 2H), 3.75 (s, 3H), 3.38 (dd, *J*=7.1, 4.1 Hz, 1H), 2.83 (d, *J*=7.1 Hz, 1H), 2.79 (s, 3H), 2.55 (d, *J*=4.1 Hz, 1H); ¹³C-NMR spectrum (100 MHz): (CDCl₃, HMDSO) δ: 167.3, 139.4, 135.6, 134.0, 132.7, 129.5, 126.1, 52.8, 35.4, 32.2, 20.6; LCMS ESI⁺ (m/z): 256.2 [M+H]⁺, LCMS purity 100.00% |
| C-3324 | 1-[4-(Trifluoromethyl)phenyl]sulfonylaziridine-2-carboxamide |
| | Molecular formula: C₁₀H₉F₃N₂O₃S; Molecular weight: 294.25; Melting point: 138-140°C; ¹H-NMR spectrum (400 MHz): (DMSO-d₆, HMDSO) δ: 8.19 (d, *J*=8.3 Hz, 2H), 8.08 (d, *J*=8.3 Hz, 2H), 7.86 (br s, 1H), 7.45 (br s, 1H), 3.36 (dd, *J*=7.2, 4.5 Hz, 1H), 2.76 (d, *J*=7.2, 1H), 2.56 (d, *J*=4.5, 1H); ¹³C-NMR spectrum (100 MHz): (DMSO-d₆, HMDSO) δ: 166.6, 141.2, 133.9 (q, *J*=32.7 Hz), 129.2, 127.3 (q, *J*=3.6 Hz), 123.6 (q, *J*=273.4 Hz), 37.5, 32.0; LCMS ESI⁺ (m/z): 295.2 [M+H]⁺, LCMS purity 100.00% |
| C-3326 | Methyl 1-[[5-(dimethylamino)-1-naphthyl]sulfonyl]aziridine-2-carboxylate |
| | Molecular formula: C₁₆H₁₈N₂O₄S; Molecular weight: 334.39; Melting point: 117-119°C; ¹H-NMR spectrum (400 MHz): (CDCl₃, HMDSO) δ: 8.63 (dt, *J*=8.5, 1.1 Hz, 1H), 8.55 (dt, *J*=8.7, 0.9 Hz, 1H), 8.22 (dd, *J*=7.4, 1.3 Hz, 1H), 7.61 (dd, *J*=8.7, 7.6 Hz, 1H), 7.54 (dd, *J*=8.5, 7.4 Hz, 1H), 7.21 (dd, *J*=7.6, 0.9 Hz, 1H), 3.74 (s, 3H), 3.49 (dd, *J*=7.1, 4.2 Hz, 1H), ), 2.89 (s, 6H), 2.87 (d, *J*=7.1 Hz, 1H), 2.58 (d, *J*=4.2 Hz, 1H); ¹³C-NMR spectrum (100 MHz): (CDCl₃, HMDSO) δ: 167.2, 151.7, 132.7, 131.9, 130.4, 129.9, 129.9, 128.7, 123.0, 120.0, 115.5, 52.8, 45.4, 35.8, 32.5; LCMS ESI⁺ (m/z): 335.1 [M+H]⁺, LCMS purity 100.00% |
| C-3327 | Methyl 1-[(6-methoxy-2-naphthyl)sulfonyl]aziridine-2-carboxylate |
| | Molecular formula: C₁₅H₁₅NO₅S; Molecular weight: 321.35; Melting point: 97-99°C; ¹H-NMR spectrum (400 MHz): (CDCl₃, HMDSO) δ: 8.42-8.44 (m, 1H), 7.85-7.93 (m, 3H), 7.28 (dd, *J=9.0,* 2.5 Hz, 1H), 7.20 (d, *J*=2.5 Hz, 1H), 3.97 (s, 3H), 3.73 (s, 3H), 3.41 (dd, *J*=7.1, 4.1 Hz, 1H), 2.82 (d, *J*=7.1 Hz, 1H), 2.58 (d, *J*=4.1 Hz, 1H); ¹³C-NMR spectrum (100 MHz): (CDCl₃, HMDSO) δ: 167.2, 160.4, 137.4, 131.3, 131.0, 129.8, 128.1, 127.3, 123.6, 120.9, 105.9, 55.5, 52.9, 35.7, 32.1; LCMS ESI⁺ (m/z): 322.2 [M+H]⁺, LCMS purity 95.29% |
| C-3329 | Methyl 1-[(6-methyl-2-naphthyl)sulfonyl]aziridine-2-carboxylate |
| | Molecular formula: C₁₅H₁₅NO₄S; Molecular weight: 305.07; Melting point: 91-93°C; ¹H-NMR spectrum (400 MHz): (CDCl₃, HMDSO) δ: 8.63-8.66 (m, 1H), 8.21 (dd, *J*=7.4, 1.3 Hz, 1H), 8.9 (dt, *J*=8.2, 1.2 Hz, 1H), 7.84 (d, *J*=1.4 Hz, 1H), 7.48 (dd, *J*=8.2, 7.4 Hz, 1H), 7.46 (dd, *J*=8.4, 1.6 Hz, 1H), 3.71 (s, 3H), 3.50 (dd, *J*=7.1, 4.1 Hz, 1H), 2.88 (d, *J*=7.1 Hz, 1H), 2.61 (s, 3H), 2.59 (d, *J*=4.1 Hz, 1H); ¹³C-NMR spectrum (100 MHz): (CDCl₃, HMDSO) δ: 167.2, 138.9, 135.4, 132.5, 131.7, 130.0, 129.4, 129.2, 128.5, 124.4, 123.0, 52.8, 35.9, 32.5, 22.4; LCMS ESI⁺ (m/z): 306.3 [M+H]⁺, LCMS purity 98.42% |
| C-3332 | Methyl 1-(4-ethylphenyl)sulfonylaziridine-2-carboxylate |
| | Molecular formula: C₁₂H₁₃NO₄S; Molecular weight: 269.07; Melting point: oil; ¹H-NMR spectrum (400 MHz): (CDCl₃, HMDSO) δ: 7.87 (d, *J*=8.4 Hz, 2H), 7.38 (d, *J*=8.4 Hz, 2H), 3.74 (s, 3H), 3.35 (dd, *J*=7.1, 4.1 Hz, 1H), 2.71-2.78 (m, 3H), 2.56 (d, *J*=4.1 Hz, 1H), 1.27 (t, *J*=7.6 Hz, 3H); ¹³C-NMR spectrum (100 MHz): (CDCl₃, HMDSO) δ: 167.2, 151.3, 134.1, 128.7, 128.3, 52.9, 35.6, 32.0, 28.9, 15.0; LCMS ESI⁺ (m/z): 270.3 [M+H]⁺, LCMS purity 97.57% |
| C-3336 | Methyl 1-[(4-methyl-1-naphthyl)sulfonyl]aziridine-2-carboxylate |
| | Molecular formula: C₁₅H₁₅NO₄S; Molecular weight: 305.07; Melting point: 96-98°C; ¹H-NMR spectrum (400 MHz): (CDCl₃, HMDSO) δ: 8.93 (d, *J*=8.8 Hz, 1H), 8.13 (d, *J*=7.6 Hz, 1H), 8.11 (dd, *J*=8.0, 1.1 Hz, 1H), 7.72 (ddd, *J*=8.3, 6.9 Hz, 1.4 Hz, 1H), 7.66 (ddd, *J*=8.3, 6.9 Hz, 1.4 Hz, 1H), 7.41 (dd, *J*=7.6, 0.9 Hz, 1H), 3.70 (s, 3H), 3.46 (dd, *J*=7.1, 4.1 Hz, 1H), 2.86 (d, *J*=7.1 Hz, 1H), 2.79 (s, 3H), 2.57 (d, *J*=4.1 Hz, 1H); ¹³C-NMR spectrum (100 MHz): (CDCl₃, HMDSO) δ: 167.2, 143.2, 133.3, 130.7, 129.8, 129.0, 128.2, 127.0, 126.1, 124.8, 124.7, 52.8, 35.8, 32.4, 20.2; LCMS ESI⁺ (m/z): 306.3 [M+H]⁺, LCMS purity 100.00% |
| C-3342 | N,N-Dimethyl-1-(p-tolylsulfonyl)aziridine-2-carboxamide |
| | Molecular formula: C₁₂H₁₆N₂O₃S; Molecular weight: 268.33; Melting point: oil; ¹H-NMR spectrum (400 MHz): (CDCl₃, HMDSO) δ: 7.85 (d, *J*=8.4 Hz, 2H), 7.35 (d, *J*=8.4 Hz, 2H), 3.59 (dd, *J*=6.9, 4.2 Hz, 1H), 3.22 (s, 3H), 2.97 (s, 3H), 2.68 (d, *J*=4.2 Hz, 1H), 2.64 (d, *J*=6.9 Hz, 1H), 2.45 (s, 3H); ¹³C-NMR spectrum (100 MHz): (CDCl₃, HMDSO) δ: 164.9, 145.1, 134.2, 129.8, 128.1, 37.2, 36.0, 35.2, 32.4, 21.6; LCMS ESI⁺ (m/z): 269.2 [M+H]⁺, LCMS purity 100.00% |
| C-3343 | Methyl 1-[4-(dimethylamino)phenyl]sulfonylaziridine-2-carboxylate |
| | Molecular formula: C₁₂H₁₆N₂O₄S; Molecular weight: 284.33; Melting point: 84-86°C; ¹H-NMR spectrum (400 MHz): (CDCl₃, HMDSO) δ: 7.74 (d, *J*=9.1 Hz, 2H), 6.68 (d, *J*=9.1 Hz, 2H), 3.73 (s, 3H), 3.25 (dd, *J*=7.1, 4.1 Hz, 1H), 3.07 (s, 6H), 2.67 (d, *J*=7.1 Hz, 1H), 2.50 (d, *J*=4.1 Hz, 1H); ¹³C-NMR spectrum (100 MHz): (CDCl₃, HMDSO) δ: 167.6, 153.7, 130.1, 121.1, 110.9, 52.7, 40.0, 35.5, 31.8; LCMS ESI⁺ (m/z): 285.2 [M+H]⁺, LCMS purity 100.00% |
| C-3346 | Methyl 1-(2-furylsulfonyl)aziridine-2-carboxylate |
| | Molecular formula: C₈H₉NO₅S; Molecular weight: 231.02; Melting point: oil; ¹H-NMR spectrum (400 MHz): (CDCl₃, HMDSO) δ: 7.67 (dd, *J*=1.8, 1.0 Hz, 1H), 7.23 (dd, *J*=3.5, 1.0 Hz, 1H), 6.58 (dd, *J*=3.5, 1.8 Hz, 1H), 3.78 (s, 3H), 3.42 (dd, *J*=7.1, 4.2 Hz, 1H), 2.85 (d, *J*=7.1 Hz, 1H), 2.68 (d, *J*=4.2 Hz, 1H); ¹³C-NMR spectrum (100 MHz): (CDCl₃, HMDSO) δ: 166.8, 147.9, 145.5, 111.6, 53.0, 35.9, 32.3; LCMS ESI⁺ (m/z): 232.1 [M+H]⁺, LCMS purity 99.01% |
| C-3350 | Methyl 1-[(2-oxo-1H-quinolin-6-yl)sulfonyl]aziridine-2-carboxylate |
| | Molecular formula: C₁₃H₁₂N₅O₅S; Molecular weight: 308.31; Melting point: 139-141°C; ¹H-NMR spectrum (400 MHz): (CDCl₃, HMDSO) δ: 12.78 (br s, 1H), 8.24 (d, *J*=2.0 Hz, 1H), 8.05 (dd, *J*=8.7, 2.0 Hz, 1H), 7.90 (d, *J*=9.6 Hz, 1H), 7.67 (d, *J*=8.7 Hz, 1H), 6.84 (d, *J*=9.6 Hz, 1H), 3.74 (s, 3H), 3.41 (dd, *J*=7.1, 4.1 Hz, 1H), 2.84 (d, *J*=7.1 Hz, 1H), 2.61 (d, *J*=4.1 Hz, 1H); ¹³C-NMR spectrum (100 MHz): (CDCl₃, HMDSO) δ: 167.0, 164.6, 142.0, 140.7, 131.1, 129.4, 129.1, 123.4, 119.4, 117.1, 53.0, 35.9, 32.2; LCMS ESI⁺ (m/z): 309.1 [M+H]⁺, LCMS purity 96.88% |
| C-3353 | Methyl 1-(2,4-difluorophenyl)sulfonylaziridine-2-carboxylate |
| | Molecular formula: C₁₀H₉F₂NO₄S; Molecular weight: 277.24; Melting point: 40-42°C; ¹H-NMR spectrum (400 MHz): (CDCl₃, HMDSO) δ: 7.99 (ddd, *J*=7.9, 7.9, 7.9 Hz, 1H), 7.03-7.08 (m, 1H), 6.97-7.03 (m, 1H), 3.77 (s, 3H), 3.52 (ddd, *J*=7.2, 4.3, 0.9 Hz, 1H), 2.97 (dd, *J*=7.2, 1.2 Hz, 1H), 2.69 (d, *J*=4.3 Hz, 1H); ¹³C-NMR spectrum (100 MHz): (CDCl₃, HMDSO) δ: 166.9, 166.7 (dd, *J*=260.0, 11.6 Hz,), 160.5 (dd, *J*=261.0, 12.9 Hz,), 132.4 (d, *J*=10.8 Hz,), 122.1 (dd, *J*=14.5, 3.4 Hz,), 112.2 (dd, *J*=22.3, 3.5 Hz,), 106.0 (t, *J*=25.5 Hz,), 53.0, 36.1, 32.6; LCMS ESI⁺ (m/z): 276.3 [M+H]⁺, LCMS purity 98.27% |
| C-3355 | Methyl 1-(2-oxochromen-6-yl)sulfonylaziridine-2-carboxylate |
| | Molecular formula: C₁₃H₁₁NO₆S; Molecular weight: 309.29; Melting point: 42-44°C; ¹H-NMR spectrum (400 MHz): (CDCl₃, HMDSO) δ: 12.78 (br s, 1H), 8.24 (d, *J*=2.0 Hz, 1H), 8.05 (dd, *J*=8.7, 2.0 Hz, 1H), 7.90 (d, *J*=9.6 Hz, 1H), 7.67 (d, *J*=8.7 Hz, 1H), 6.84 (d, *J*=9.6 Hz, 1H), 3.74 (s, 3H), 3.41 (dd, *J*=7.1, 4.1 Hz, 1H), 2.84 (d, *J*=7.1 Hz, 1H), 2.61 (d, *J*=4.1 Hz, 1H); ¹³C-NMR spectrum (100 MHz): (CDCl₃, HMDSO) δ: 166.8, 159.0, 157.3, 142.1, 133.4, 131.0, 128.7, 119.1, 118.7, 118.2, 53.0, 36.0, 32.3; LCMS ESI⁺ (m/z): 310.1 [M+H]⁺, LCMS purity 100.00% |
| C-3357 | 1-[4-(Dimethylamino)phenyl]sulfonyl-N,N-dimethyl-aziridine-2-carboxamide |
| | Molecular formula: C₁₃H₁₉N₃O₃S; Molecular weight: 297.37; Melting point: 144-146°C; ¹H-NMR spectrum (400 MHz): (CDCl₃, HMDSO) δ: 7.74 (d, *J*=9.2 Hz, 2H), 6.68 (d, *J*=9.2 Hz, 2H), 3.49 (dd, *J*=6.9, 4.2 Hz, 1H), 3.23 (s, 3H), 3.06 (s, 6H), 2.97 (s, 3H), 2.61 (d, *J*=4.2 Hz, 1H), 2.55 (d, *J*=6.9 Hz, 1H); ¹³C-NMR spectrum (100 MHz): (CDCl₃, HMDSO) δ: 165.3, 153.6, 130.0, 121.4, 110.8, 40.0, 37.3, 36.0, 35.4, 31.9; LCMS ESI⁺ (m/z): 298.2 [M+H]⁺, LCMS purity 100.00% |
| C-3362 | 1-(4-Butylphenyl)sulfonyl-N,N-dimethyl-aziridine-2-carboxamide |
| | Molecular formula: C₁₅H₂₂N₂O₃S; Molecular weight: 310.41; Melting point: oil; ¹H-NMR spectrum (400 MHz): (CDCl₃, HMDSO) δ: 7.86 (d, *J*=8.4 Hz, 2H), 7.35 (d, *J*=8.4 Hz, 2H), 3.59 (dd, *J*=6.9, 4.2 Hz, 1H), 3.22 (s, 3H), 2.98 (s, 3H), 2.69 (t, *J*=7.6 Hz, 2H), 2.68 (d, *J*=4.2 Hz, 1H), 2.65 (d, *J*=6.9 Hz, 1H), 1.57-1.66 (m, 2H), 1.35 (sextet, *J*=7.5 Hz, 2H), 0.93 (d, *J*=7.4 Hz, 3H); ¹³C-NMR spectrum (100 MHz): (CDCl₃, HMDSO) δ: 164.9, 150.0, 134.3, 129.2, 128.2, 37.2, 36.0, 35.6, 35.2, 33.1, 32.5, 22.2, 13.8; LCMS ESI⁺ (m/z): 311.2 [M+H]⁺, LCMS purity 100.00% |
| C-3364 | 1-(4-Isopropylphenyl)sulfonyl-N,N-dimethyl-aziridine-2-carboxamide |
| | Molecular formula: C₁₄H₂₀N₂O₃S; Molecular weight: 296.38; Melting point: oil; ¹H-NMR spectrum (400 MHz): (CDCl₃, HMDSO) δ: 7.88 (d, *J*=8.5 Hz, 2H), 7.40 (d, *J*=8.5 Hz, 2H), 3.55 (dd, *J*=6.9, 4.2 Hz, 1H), 3.23 (s, 3H), 3.00 (septet, *J*=6.9 Hz, 1H), 2.98 (s, 3H), 2.69 (d, *J*=4.2 Hz, 1H), 2.65 (d, *J*=6.9 Hz, 1H), 1.28 (d, *J*=6.9 Hz, 6H); ¹³C-NMR spectrum (100 MHz): (CDCl₃, HMDSO) δ: 164.9, 155.8, 134.5, 128.3, 127.3, 37.2, 36.0, 35.2, 34.3, 32.6, 23.6; LCMS ESI⁺ (m/z): 297.2 [M+H]⁺, LCMS purity 100.00% |
| C-3365 | N,N-Dimethyl-1-(1-naphthylsulfonyl)aziridine-2-carboxamide |
| | Molecular formula: C₁₅H₁₆N₂O₃S; Molecular weight: 304.36; Melting point: 110-112°C; ¹H-NMR spectrum (400 MHz): (CDCl₃, HMDSO) δ: 8.87 (ddd, *J*=8.5, 1.8, 0.9 Hz, 1H), 8.25 (dd, *J*=7.4, 1.3 Hz, 1H), 8.15 (dt, *J*=8.2, 1.2 Hz, 1H), 7.95 (ddd, *J*=8.2, 1.3, 0.6 Hz, 1H), 7.72 (ddd, *J*=8.5, 6.9, 1.4 Hz, 1H), 7.63 (ddd, *J*=8.2, 6.9, 1.2 Hz, 1H), 7.56 (dd, *J*=8.2, 7.4 Hz, 1H), 3.75 (dd, *J*=6.9, 4.3 Hz, 1H), 3.20 (s, 3H), 2.97 (s, 3H), 2.74 (d, *J*=6.9 Hz, 1H), 2.69 (d, *J*=4.3 Hz, 1H); ¹³C-NMR spectrum (100 MHz): (CDCl₃, HMDSO) δ: 164.9, 135.6, 134.2, 132.8, 129.7, 129.0, 128.7, 128.7, 127.2, 125.7, 123.9, 37.2, 36.0, 35.8, 32.7; LCMS ESI⁺ (m/z): 305.2 [M+H]⁺, LCMS purity 96.57% |
| C-3366 | N,N-Dimethyl-1-[(4-methyl-1-naphthyl)sulfonyl]aziridine-2-carboxamide |
| | Molecular formula: C₁₆H₁₈N₂O₃S; Molecular weight: 318.39; Melting point: 120-122°C; ¹H-NMR spectrum (400 MHz): (CDCl₃, HMDSO) δ: 8.92 (ddd, *J*=8.3, 1.4, 0.7 Hz, 1H), 8.14 (d, *J*=7.6 Hz, 1H), 8.10 (ddd, *J*=8.3, 1.3, 0.7 Hz, 1H), 7.71 (ddd, *J*=8.3, 6.8, 1.5 Hz, 1H), 7.66 (ddd, *J*=8.3, 6.9, 1.5 Hz, 1H), 7.41 (ddd, *J*=7.6, 1.9, 0.9 Hz, 1H), 3.73 (dd, *J*=6.9, 4.3 Hz, 1H), 3.20 (s, 3H), 2.97 (s, 3H), 2.79 (s, 3H), 2.70 (d, *J*=6.9 Hz, 1H), 2.66 (d, *J*=4.3 Hz, 1H); ¹³C-NMR spectrum (100 MHz): (CDCl₃, HMDSO) δ: 164.9, 143.1, 133.3, 130.9, 129.7, 129.0, 128.2, 127.0, 126.2, 124.8, 124.7, 37.3, 35.9, 35.9, 32.6, 20.2; LCMS ESI⁺ (m/z): 319.2 [M+H]⁺, LCMS purity 98.41% |
| C-3368 | N,N-Dimethyl-1-(4-pentylphenyl)sulfonyl-aziridine-2-carboxamide |
| | Molecular formula: C₁₆H₂₄N₂O₃S; Molecular weight: 324.44; Melting point: oil; ¹H-NMR spectrum (400 MHz): (CDCl₃, HMDSO) δ: 7.85 (d, *J*=8.5 Hz, 2H), 7.30 (d, *J*=8.5 Hz, 2H), 3.59 (dd, *J*=6.9, 4.3 Hz, 1H), 3.21 (s, 3H), 2.97 (s, 3H), 2.68 (t, *J*=7.8 Hz, 2H), 2.68 (d, *J*=4.3 Hz, 1H), 2.65 (d, *J*=6.9 Hz, 1H), 1.63 (quintet, *J*=7.5 Hz, 2H), 1.23-1.39 (m, 4H), 0.89 (t, *J*=7.0 Hz, 3H); ¹³C-NMR spectrum (100 MHz): (CDCl₃, HMDSO) δ: 164.9, 150.0, 134.4, 129.2, 128.2, 37.2, 36.0, 35.9, 35.2, 32.5, 31.3, 30.7, 22.4, 13.9; LCMS ESI⁺ (m/z): 325.2 [M+H]⁺, LCMS purity 100.00% |
| C-3369 | 1-(4-Hexylphenyl)sulfonyl-N,N-dimethyl-aziridine-2-carboxamide |
| | Molecular formula: C₁₇H₂₆N₂O₃S; Molecular weight: 338.46; Melting point: oil; ¹H-NMR spectrum (400 MHz): (CDCl₃, HMDSO) δ: 7.86 (d, *J*=8.5 Hz, 2H), 7.34 (d, *J*=8.5 Hz, 2H), 3.59 (dd, *J*=6.9, 4.2 Hz, 1H), 3.21 (s, 3H), 2.97 (s, 3H), 2.68 (t, *J*=7.8 Hz, 2H), 2.68 (d, *J*=4.2 Hz, 1H), 2.65 (d, *J*=6.9 Hz, 1H), 1.62 (quintet, *J*=7.5 Hz, 2H), 1.23-1.37 (m, 6H), 0.88 (t, *J*=7.0 Hz, 3H); ¹³C-NMR spectrum (100 MHz): (CDCl₃, HMDSO) δ: 165.0, 150.0, 134.3, 129.2, 128.2, 37.2, 36.0, 35.9, 35.2, 32.5, 31.6, 30.9, 28.8, 22.5, 14.0; LCMS ESI⁺ (m/z): 339.2 [M+H]⁺, LCMS purity 95.86% |
| C-3371 | 1-[[5-(Dimethylamino)-1-naphthyl]sulfonyl]-N,N-dimethylaziridine-2-carboxamide |
| | Molecular formula: C₁₇H₂₁N₃O₃S; Molecular weight: 347.43; Melting point: oil; ¹H-NMR spectrum (400 MHz): (CDCl₃, HMDSO) δ: 8.62 (dt, *J*=8.5, 1.1 Hz, 1H), 8.55 (dt, *J*=8.7, 0.9 Hz, 1H), 8.23 (dd, *J*=7.4, 1.3 Hz, 1H), 7.60 (dd, *J*=8.7, 7.6 Hz, 1H), 7.53 (dd, *J*=8.5, 7.4 Hz, 1H), 7.21 (dd, *J*=7.6, 0.9 Hz, 1H), 3.74 (dd, *J*=6.9, 4.3 Hz, 1H), 3.20 (s, 3H), 2.96 (s, 3H), 2.88 (s, 6H), 2.73 (d, *J*=6.9 Hz, 1H), 2.68 (d, *J*=4.3 Hz, 1H); ¹³C-NMR spectrum (100 MHz): (CDCl₃, HMDSO) δ: 164.9, 151.6, 132.9, 131.8, 130.4, 129.9, 129.7, 128.7, 122.9, 120.1, 115.5, 45.4, 37.3, 35.9, 35.8, 32.7; LCMS ESI⁺ (m/z): 348.2 [M+H]⁺, LCMS purity 95.88 % |
| C-3373 | N,N-Dimethyl-1-(2-naphthylsulfonyl)aziridine-2-carboxamide |
| | Molecular formula: C₁₃H₁₆N₂O₃S; Molecular weight: 304.36; Melting point: oil |
| | ¹H-NMR spectrum (400 MHz): (CDCl₃) δ: 8.54-8.52 (m, 1H), 8.04-7.90 (m, 4H), 7.73-7.59 (m, 2H), 3.67 (dd, J=6.9, 4.3 Hz, 1H), 3.24 (s, 3H), 2.98 (s, 3H), 2.74 (d, J=6.9 Hz, 1H), 2.72 (d, J=4.3 Hz, 1H); ¹³C-NMR spectrum (100 MHz): (CDCl₃, HMDSO) δ: 165.0, 135.6, 134.3, 132.1, 129.9, 129.7, 129.6, 128.2, 127.9, 123.1, 37.4, 36.2, 35.5, 32.8 LCMS ESI+ (m/z): 305.2 [M+H]+ , LCMS purity 97.08% |
| C-3374 | N,N-Dimethyl-1-[4-(trifluoromethyl)phenyl]sulfonyl-aziridine-2-carboxamide |
| | Molecular formula: C₁₂H₁₃N₂O₃S; Molecular weight: 322.30; Melting point: oil; ¹H-NMR spectrum (400 MHz): 8.12 (d, *J*=8.4 Hz, 2H), 7.83 (d, *J*=8.4 Hz, 2H), 3.68 (dd, *J*=6.9, 4.3 Hz, 1H), 3.25 (s, 3H), 3.00 (s, 3H), 2.75 (d, *J*=4.3 Hz, 1H), 2.73 (d, *J*=6.9 Hz, 1H); ¹³C-NMR spectrum (100 MHz): (CDCl₃, HMDSO) δ: 164.4, 141.1, 135.6 (q, *J*=33.3 Hz), 128.6, 126.3 (q, *J*=3.7 Hz), 123.0 (q, *J*=273.2 Hz), 37.2, 36.0, 35.3, 33.0; LCMS ESI⁺ (m/z): 323.1 [M+H]⁺, LCMS purity 95.04.00% |
| C-3375 | Methyl 1-[[6-(dimethylamino)-1-naphthyl]sulfonyl]aziridine-2-caiboxylate |
| | Molecular formula: C₁₆H₁₈N₂O₄S; Molecular weight: 334.39; Melting point: 106-108°C ¹H-NMR spectrum (400 MHz): (CDCl₃, HMDSO) δ: 8.73 (dt, J=9.5, 0.6 Hz, 1H), 7.93 (d, J=8.3 Hz, 1H), 7.89 (dd, J=7.3, 1.2 Hz, 1H), 7.40 (dd, J=8.3, 7.3 Hz, 1H), 7.34 (dd, J=9.5, 2.6 Hz, 1H), 6.94 (d, J=2.6 Hz, 1H), 3.70 (s, 3H), 3.44 (dd, J=7.1, 4.1 Hz, 1H), ), 3.09 (s, 6H), 2.83 (d, J=7.1 Hz, 1H), 2.55 (d, J=4.2 Hz, 1H); ¹³C-NMR spectrum (100 MHz): (CDCl₃, HMDSO) δ: 167.3, 148.9, 136.2, 133.8, 132.1, 126.2, 125.6, 124.1, 121.6, 118.2, 106.2, 52.7, 40.4, 35.7, 32.3 |
| | LCMS ESI⁺ (m/z): 335.1 [M+H]⁺ , LCMS purity 100% |
| C-3376 | Methyl 1-[[6-(dimethylamino)-5-formyl-1-naphthyl]sulfonyl]aziridine-2-carboxylate |
| | Molecular formula: C₁₇H₁₈N₂O₅S; Molecular weight: 362.40; Melting point: 116-117°C; ¹H-NMR spectrum (400 MHz): (CDCl₃, HMDSO) δ: 10.30 (s, 1H), 9.49 (dt, *J*=8.7, 1.1 Hz, 1H), 9.02 (dd, *J*=9.7, 0.9 Hz, 1H), 8.05 (dd, *J*=7.4, 1.1 Hz, 1H), 7.62 (dd, *J*=8.7, 7.4 Hz, 1H), 7.53 (d, *J*=9.7 Hz, 1H), 3.72 (s, 3H), 3.47 (dd, *J*=7.1, 4.1 Hz, 1H), 3.19 (s, 6H), 2.85 (d, *J*=7.1 Hz, 1H), 2.55 (d, *J*=4.2 Hz, 1H); ¹³C-NMR spectrum (100 MHz): (CDCl₃, HMDSO) δ: 190.8, 167.1, 158.1, 133.9, 132.6, 132.0, 131.3, 127.2, 127.0, 123.7, 120.4, 116.4, 52.8, 45.9, 35.8, 32.4; LCMS ESI⁺ (m/z): 363.1 [M+H]⁺, LCMS purity 100.00% |
| C-3377 | 1-[[6-(Dimethylamino)-1-naphthyl]sulfonyl]-N,N-dimethylaziridine-2-carboxamide Molecular formula: C₁₇H₂₁N₃O₃S; Molecular weight: 347.43; Melting point: 147-149°C ¹H-NMR spectrum (400 MHz): (CDCl₃, HMDSO) δ: 8.72 (d, J=9.5 Hz, 1H), 7.90 (d, J=8.4 Hz, 1H), 7.89 (dd, J=7.3, 1.2 Hz, 1H), 7.49 (dd, J=8.2, 7.5 Hz, 1H), 7.32 (dd, J=9.5, 2.7 Hz, 1H), 6.93 (d, J=2.7 Hz, 1H), 3.67 (dd, J=6.9, 4.3 Hz, 1H), 3.14 (s, 3H), 3.08 (s, 6H), 2.94 (s, 3H), 2.71 (d, J=6.9 Hz, 1H), 2.67 (d, J=4.3 Hz, 1H); ¹³C-NMR spectrum (100 MHz): (CDCl₃, HMDSO) δ: 165.0, 148.9, 136.2, 133.7, 132.3, 126.3, 125.3, 124.1, 121.5, 118.2, 106.1, 40.3, 37.1, 35.9, 35.9, 32.3 |
| | LCMS ESI+ (m/z): 348.1 [M+H]+ , LCMS purity 100% |
| C-3380 | 1-(4-Hexylphenyl)sulfonyl-N-methyl-aziridine-2-carboxamide |
| | Molecular formula: C₁₆H₂₄N₂O₃S; Molecular weight: 324.44; Melting point: 84-86°C; ¹H-NMR spectrum (400 MHz): (CDCl₃, HMDSO) δ: 7.83 (d, *J*=8.5 Hz, 2H), 7.38 (d, *J*=8.5 Hz, 2H), 6.15 (br s, 1H), 3.28 (dd, *J*=7.7, 4.2 Hz, 1H), 2.75 (d, *J*=4.9 Hz, 3H), 2.74 (d, *J*=7.7 Hz, 1H), 2.71 (t, *J*=7.8 Hz, 2H), 2.36 (d, *J*=4.2 Hz, 1H), 1.64 (quintet, *J*=7.5 Hz, 2H), 1.25-1.39 (m, 6H), 0.89 (t, *J*=7.0 Hz, 3H); ¹³C-NMR spectrum (100 MHz): (CDCl₃, HMDSO) δ: 166.2, 150.4, 133.4, 129.4, 128.2, 37.9, 36.0, 33.4, 31.5, 30.8, 28.8, 25.9, 22.5, 14.0; LCMS ESI⁺ (m/z): 325.1 [M+H]⁺, LCMS purity 97.38% |
| C-3383 | Methyl 1-[[4-(dimethylamino)-1-naphthyl]sulfonyl]aziridine-2-caiboxylate |
| | Molecular formula: C₁₆H₁₈N₂O₄S; Molecular weight: 334.39; Melting point: oil; ¹H-NMR spectrum (400 MHz): (CDCl₃, HMDSO) δ: 8.86 (ddd, *J*=8.6, 1.3, 0.7 Hz, 1H), 8.23 (ddd, *J*=8.6, 1.4, 0.7 Hz, 1H), 8.11 (d, *J*=8.3 Hz, 1H), 7.67 (ddd, *J*=8.6, 6.9, 1.4 Hz, 1H), 7.56 (ddd, *J*=8.6, 6.9, 1.3 Hz, 1H), 6.97 (d, *J*=8.3 Hz, 1H), 3.70 (s, 3H), 3.44 (dd, *J*=7.1, 4.1 Hz, 1H), 3.01 (s, 6H), 2.83 (d, *J*=7.1 Hz, 1H), 2.54 (d, *J*=4.1 Hz, 1H); ¹³C-NMR spectrum (100 MHz): (CDCl₃, HMDSO) δ: 167.4, 157.5, 131.3, 130.8, 128.3, 128.2, 125.9, 125.7, 125.3, 124.3, 110.7, 52.7, 44.6, 35.7, 32.4; LCMS ESI⁺ (m/z): 335.1 [M+H]⁺, LCMS purity 100.00% |
| C-3384 | 1-(4-Cyclohexylphenyl)sulfonyl-N,N-dimethyl-aziridine-2-carboxamide |
| | Molecular formula: C₁₇H₂₄N₂O₃S; Molecular weight: 336.45; Melting point: oil; ¹H-NMR spectrum (400 MHz): (CDCl₃, HMDSO) δ: 7.86 (d, *J*=8.5 Hz, 2H), 7.37 (d, *J*=8.5 Hz, 2H), 3.58 (dd, *J*=6.9, 4.3 Hz, 1H), 3.21 (s, 3H), 2.97 (s, 3H), 2.68 (d, *J*=4.3 Hz, 1H), 2.65 (d, *J*=6.9 Hz, 1H), 2.54-2.63 (m, 1H), 1.80-1.92 (m, 4H), 1.72-1.80 (m, 1H), 1.32-1.48 (m, 4H), 1.19-1.32 (m, 1H); ¹³C-NMR spectrum (100 MHz): (CDCl₃, HMDSO) δ: 164.9, 154.9, 134.4, 128.2, 127.7, 44.6, 37.3, 36.0, 35.1, 34.0, 32.5, 26.6, 25.9; LCMS ESI⁺ (m/z): 337.2 [M+H]⁺, LCMS purity 95.84% |
| C-3385 | 1-[[4-(Dimethylamino)-1-naphthyl]sulfonyl]-N,N-dimethylaziridine-2-caboxamide |
| | Molecular formula: C₁₇H₂₁N₃O₃S; Molecular weight: 347.43; Melting point: 120-122°C ¹H-NMR spectrum (400 MHz): (CDCl₃, HMDSO) δ: 8.85 (ddd, *J*=8.6, 1.3, 0.6 Hz, 1H), 8.22 (ddd, *J*=8.6, 1.3, 0.6 Hz, 1H), 8.12 (d, *J*=8.2 Hz, 1H), 7.65 (ddd, *J*=8.6, 6.9, 1.4 Hz, 1H), 7.56 (ddd, *J*=8.6, 6.9, 1.4 Hz, 1H), 6.97 (d, *J*=8.2 Hz, 1H), 3.69 (dd, *J*=6.9, 4.3 Hz, 1H), 3.20 (s, 3H), 3.01 (s, 6H), 2.96 (s, 3H), 2.68 (d, *J*=6.9 Hz, 1H), 2.64 (d, *J*=4.3 Hz, 1H); ¹³C-NMR spectrum (100 MHz): (CDCl₃, HMDSO) δ: 165.2, 157.4, 131.0, 130.8, 128.3, 128.2, 126.0, 125.7, 125.3, 124.6, 110.6, 44.6, 37.3, 35.9, 35.8, 32.5 |
| | LCMS ESI⁺ (m/z): 348.1 [M+H]⁺, LCMS purity 100% |
| C-3389 | 1-(4-Hexylphenyl)sulfonylaziridine-2-carboxamide |
| | Molecular formula: C₁₅H₂₂N₂O₃S; Molecular weight: 310.41; Melting point: 103-105°C; ¹H-NMR spectrum (400 MHz): (DMSO-d₆, HMDSO) δ: 7.84 (d, *J*=8.4 Hz, 2H), 7.81 (br s, 1H), 7.50 (d, *J*=8.4 Hz, 2H), 7.41 (br s, 1H), 3.23 (dd, *J*=7.2, 4.3 Hz, 1H), 2.69 (t, *J*=7.7 Hz, 2H), 2.62 (d, *J*=7.2 Hz, 1H), 2.46 (d, *J*=4.3 Hz, 1H), 1.60 (quintet, *J*=7.4 Hz, 2H), 1.22-1.33 (m, 6H), 0.85 (t, *J*=7.0 Hz, 3H); ¹³C-NMR spectrum (100 MHz): (DMSO-d₆, HMDSO) δ: 167.0, 150.0, 134.5, 129.8, 128.2, 37.1, 35.4, 31.6, 31.4, 30.9, 28.7, 22.4, 14.3; LCMS ESI⁺ (m/z): 311.1 [M+H]⁺, LCMS purity 100.00% |
| C-3390 | Methyl 1-[[6-(dimethylamino)-5-formyl-2-naphthyl]sulfonyl]aziridine-2-carboxylate |
| | Molecular formula: C₁₇H₁₈N₂O₅S; Molecular weight: 362.40; Melting point: 147-149°C |
| | ¹H-NMR spectrum (400 MHz): (CDCl3, HMDSO) δ: 10.27 (s, 1H), 9.22 (d, J=9.1 Hz, 1H), 8.35 (d, J=2.0 Hz, 1H), 7.96 (dd, J=9.1, 2.0 Hz, 1H), 7.94 (d, J=9.1 Hz, 1H), 7.41 (d, J=9.1 Hz, 1H), 3.72 (s, 3H), 3.39 (dd, J=7.1, 4.1 Hz, 1H), 3.24 (s, 6H), 2.81 (d, J=7.1 Hz, 1H), 2.58 (d, J=4.1 Hz, 1H); ¹³C-NMR spectrum (100 MHz): (CDCl3, HMDSO) δ: 190.1, 167.2, 159.5, 135.9, 135.7, 131.1, 129.7, 126.5, 126.1, 125.1, 119.6, 115.2, 52.9, 45.7, 35.8, 32.0 LCMS ESI⁺ (m/z): 363.1 [M+H]⁺ , LCMS purity 96.10% |
| C-3391 | 1-[[6-(Dimethylamino)-5-formyl-2-naphthyl]sulfonyl]-N,N-dimethylaziridine-2-carboxamide |
| | Molecular formula: C₁₈H₂₁N₃O₄S; Molecular weight: 375.44; Melting point: 58-60°C; ¹H-NMR spectrum (400 MHz): (CDCl₃, HMDSO) δ: 10.27 (s, 1H), 9.21 (dt, *J*=9.2, 0.6 Hz, 1H), 8.35 (d, *J*=2.1 Hz, 1H), 7.97 (dd, *J*=9.2, 2.1 Hz, 1H), 7.94 (d, *J*=9.2 Hz, 1H), 7.41 (d, *J*=9.2 Hz, 1H), 3.64 (dd, *J*=6.9, 4.3 Hz, 1H), 3.24 (s, 3H), 3.23 (s, 6H), 2.97 (s, 3H), 2.70 (d, *J*=6.9 Hz, 1H), 2.69 (d, *J*=4.3 Hz, 1H); ¹³C-NMR spectrum (100 MHz): (CDCl₃, HMDSO) δ: 190.1, 164.9, 159.5, 135.8, 135.6, 131.5, 129.5, 126.5, 126.1, 125.0, 119.6, 115,2, 45.7, 37.2, 36.0, 35.3, 32.5; LCMS ESI⁺ (m/z): 376.1 [M+H]⁺, LCMS purity 100.00% |
| C-3393 | Methyl 1-[[5-chloro-6-(methylamino)-2-naphthyl]sulfonyl]aziridine-2-carboxylate |
| | Molecular formula: C₁₅H₁₅ClN₂O₄S; Molecular weight: 354.81; Melting point: 170-172°C; ¹H-NMR spectrum (400 MHz): (CDCl₃, HMDSO) δ: 8.36 (d, *J*=1.9 Hz, 1H), 8.12 (dt, *J*=9.1, 0.6 Hz, 1H), 7.90 (dd, *J*=9.1, 2.0 Hz, 1H), 7.83 (d, *J*=9.0 Hz, 1H), 7.19 (d, *J*=9.0 Hz, 1H), 5.01 (q, *J*=5.1 Hz, 1H), 3.72 (s, 3H), 3.39 (dd, *J*=7.1, 4.1 Hz, 1H), 3.09 (d, *J*=5.1 Hz, 3H), 2.81 (d, *J*=7.1 Hz, 1H), 2.56 (d, *J*=4.1 Hz, 1H); ¹³C-NMR spectrum (100 MHz): (CDCl₃, HMDSO) δ: 167.3, 145.4, 133.9, 130.5, 129.7, 129.1, 125.3, 124.4, 123.3, 114.3, 110.5, 52.8, 35.7, 32.0, 30.3; LCMS ESI⁺ (m/z): 355.0 [M+H]⁺, LCMS purity 100.00% |
| C-3397 | Methyl 1-[[6-(dimethylamino)-2-naphthyl]sulfonyl]aziridine-2-caiboxylate |
| | Molecular formula: C₁₆H₁₈N₂O₄S; Molecular weight: 334.39; Melting point: 156-158°C; ¹H-NMR spectrum (400 MHz): (CDCl₃, HMDSO) δ: 8.31 (d, *J*=1.9 Hz, 1H), 7.92 (d, *J*=9.2 Hz, 1H), 7.77 (dd, *J*=8.8, 1.9 Hz, 1H), 7.71 (d, *J*=8.8 Hz, 1H), 7.22 (dd, *J*=9.2, 2.6 Hz, 1H), 6.88 (d, *J*=2.6 Hz, 1H), 3.72 (s, 3H), 3.36 (dd, *J*=7.1, 4.1 Hz, 1H), 3.13 (s, 6H), 2.78 (d, *J*=7.1 Hz, 1H), 2.56 (d, *J*=4.1 Hz, 1H); ¹³C-NMR spectrum (100 MHz): (CDCl₃, HMDSO) δ: 167.4, 150.7, 137.7, 130.5, 130.0, 128.1, 127.0, 124.4, 123.4, 116.3, 104.9, 52.8, 40.3, 35.6, 32.0; LCMS ESI⁺ (m/z): 335.1 [M+H]⁺, LCMS purity 100.00% |
| C-3398 | 1-[[6-(Dimethylamino)-2-naphthyl]sulfonyl]-N,N-dimethylaziridine-2-carboxamide |
| | Molecular formula: C₁₇H₂₁N₃O₃S; Molecular weight: 347.43; Melting point: foam; ¹H-NMR spectrum (400 MHz): (CDCl₃, HMDSO) δ: 8.31 (d, *J*=2.0 Hz, 1H), 7.79 (d, *J*=9.2 Hz, 1H), 7.78 (dd, *J*=8.7, 2.0 Hz, 1H), 7.71 (d, *J*=8.7 Hz, 1H), 7.21 (dd, *J*=9.2, 2.5 Hz, 1H), 6.87 (d, *J*=2.5 Hz, 1H), 3.59 (dd, *J*=6.9, 4.3 Hz, 1H), 3.21 (s, 3H), 3.12 (s, 6H), 2.96 (s, 3H), 2.68 (d, *J*=4.3 Hz, 1H), 2.67 (d, *J*=6.9 Hz, 1H); ¹³C-NMR spectrum (100 MHz): (CDCl₃, HMDSO) δ: 165.1, 150.6, 137.6, 130.4, 129.8, 128.5, 127.0, 124.4, 123.4, 117.0, 105.0, 40.3, 37.3, 36.0, 35.4, 32.3; LCMS ESI⁺ (m/z): 348.1 [M+H]⁺, LCMS purity 96.37% |
| C-3399 | Methyl 1-[[5-(dimethylamino)-2-naphthyl]sulfonyl]aziridine-2-carboxylate |
| | Molecular formula: C₁₆H₁₈N₂O₄S; Molecular weight: 334.39; Melting point: 85-87°C ¹H-NMR spectrum (400 MHz): (CDCl3, HMDSO) δ: 8.47 (d, J=2.0 Hz, 1H), 8.40 (dt, J=9.0, 0.7 Hz, 1H), 7.93 (dd, J=9.0, 2.0 Hz, 1H), 7.63 (dt, J=8.2, 1.0 Hz, 1H), 7.54 (dd, J=8.2, 7.6 Hz, 1H), 7.25 (dd, J=7.6, 1.0 Hz, 1H), 3.73 (s, 3H), 3.43 (dd, J=7.1, 4.1 Hz, 1H), 2.90 (s, 6H), 2.84 (d, J=7.1 Hz, 1H), 2.60 (d, J=4.1 Hz, 1H); ¹³C-NMR spectrum (100 MHz): (CDCl3, HMDSO) δ: 167.2, 151.1, 133.6, 133.5, 131.1, 130.2, 127.8, 126.2, 123.9, 121.9, 117.4, 52.9, 45.1, 35.8, 32.1 |
| | LCMS ESI⁺ (m/z): 335.1 [M+H]⁺ , LCMS purity 100% |
| C-3400 | 1-[[5-(Dimethylamino)-2-naphthyl]sulfonyl]-N,N-dimethylaziridine-2-carboxamide |
| | Molecular formula: C₁₇H₂₁N₃O₃S; Molecular weight: 347.43; Melting point: 96-98°C; ¹H-NMR spectrum (400 MHz): (CDCl₃, HMDSO) δ: 8.47 (d, *J*=1.9 Hz, 1H), 8.39 (dt, *J*=9.0, 0.7 Hz, 1H), 7.94 (dd, *J*=9.0, 1.9 Hz, 1H), 7.62 (dt, *J*=8.2, 1.0 Hz, 1H), 7.53 (dd, *J*=8.2, 7.6 Hz, 1H), 7.24 (dd, *J*=7.6, 1.0 Hz, 1H), 3.67 (dd, *J*=6.9, 4.3 Hz, 1H), 3.25 (s, 3H), 2.98 (s, 3H), 2.90 (s, 6H), 2.73 (d, *J*=6.9 Hz, 1H), 2.72 (d, *J*=4.3 Hz, 1H); ¹³C-NMR spectrum (100 MHz): (CDCl₃, HMDSO) δ: 167.9, 151.1, 133.9, 133.4, 131.0, 130.0, 127.8, 126.1, 123.8, 122.0, 117.3, 45.1, 37.3, 36.0, 35.3, 32.6; LCMS ESI⁺ (m/z): 348.2 [M+H]⁺, LCMS purity 100.00% |
| C-3402 | 1-(2,4-Difluorophenyl)sulfonylaziridine-2-carboxamide |
| | Molecular formula: C₉H₈F₂NO₃S; Molecular weight: 262.23; Melting point: 141-143°C; ¹H-NMR spectrum (400 MHz): (CDCl₃, HMDSO) δ: 7.99 (dt, *J*=8.5, 6.2 Hz, 1H), 7.87 (s, 1H), 7.66-7.74 (m, 1H), 7.47 (s, 1H), 7.36-7.43 (m, 1H), 3.38 (dd, *J*=7.2, 4.5 Hz, 1H), 2.77 (d, *J*=7.2 Hz, 1H), 2.59 (d, *J*=4.5 Hz, 1H); ¹³C-NMR spectrum (100 MHz): (CDCl₃, HMDSO) δ: 166.6, 166.7 (dd, *J*=256.9, 12.1 Hz,), 160.5 (dd, *J*=258.8, 13.7 Hz,), 132.8 (d, *J*=11*.*1 Hz,), 122.2 (dd, *J*=14.6, 3.7 Hz,), 113.3 (dd, *J*=22.4, 3.4 Hz,), 107.0 (t, *J*=26.4 Hz,), 37.3, 32.3; LCMS ESI⁺ (m/z): 263.1 [M+H]⁺, LCMS purity 100.00% |
| C-3403 | 1-(2,4-Difluorophenyl)sulfonyl-N,N-dimethyl-aziridine-2-carboxamide |
| | Molecular formula: C₁₁H₁₂F₂NO₃S; Molecular weight: 290.29; Melting point: oil; ¹H-NMR spectrum (400 MHz): (CDCl₃, HMDSO) δ: 7.99 (ddd, *J*=8.8, 7.8, 6.0 Hz, 1H), 6.96-7.06 (m, 2H), 3.26 (s, 3H), 2.99 (s, 3H), 3.71 (dd, *J*=7.0, 4.4 Hz, 1H), 2.88 (d, *J*=7.0 Hz, 1H), 2.81 (d, *J*=4.4 Hz, 1H); ¹³C-NMR spectrum (100 MHz): (CDCl₃, HMDSO) δ: 166.7 (dd, *J*=259.4, 11.6 Hz,), 164.5, 160.5 (dd, *J*=260.8, 13.1 Hz,), 132.3 (d, *J*=10.9 Hz,), 122.3 (dd, *J*=14.6, 3.8 Hz,), 112.1 (dd, *J*=22.2, 3.4 Hz,), 106.0 (t, *J*=25.6 Hz,), 37.2, 36.1, 35.1, 33.4; LCMS ESI⁺ (m/z): 291.1 [M+H]⁺, LCMS purity 94.31% |
| C-3427 | Methyl 1-[[8-(dimethylamino)-2-naphthyl]sulfonyl]aziridine-2-carboxylate |
| | Molecular formula: C₁₆H₁₈N₂O₄S; Molecular weight: 334.39; Melting point: oil; |
| | ¹H-NMR spectrum (400 MHz): (CDCl₃, HMDSO) δ: 8.88-8.89 (m, 1H), 7.96 (dd, *J*=8.8, 0.7 Hz, 1H), 7.92 (dd, *J*=8.8, 1.8 Hz, 1H), 7.58 (dd, *J*=8.2, 6.7 Hz, 1H), 7.55 (ddd, *J*=8.2, 1.8, 0.7 Hz, 1H), 7.17 (dd, *J*=6.7, 1.8 Hz, 1H), 3.72 (s, 3H), 3.42 (dd, *J*=7.1, 4.1 Hz, 1H), 2.92 (s, 6H), 2.84 (d, *J*=7.1 Hz, 1H), 2.59 (d, *J*=4.1 Hz, 1H); ¹³C-NMR spectrum (100 MHz): (CDCl₃, HMDSO) δ: 167.2, 152.6, 137.1, 132.8, 129.9, 129.7, 127.6, 126.8, 122.8, 122.2, 115.7, 52.8, 45.2, 35.8, 32.1; LCMS ESI⁺ (m/z): 335.2 [M+H]⁺, LCMS purity 100.00% |
| C-3459 | 1-[[6-(Dimethylamino)-1-naphthyl]sulfonyl]aziridine-2-carboxamide |
| | Molecular formula: C₁₅H₁₇N₃O₃S; Molecular weight: 319.38; Melting point: 197-199°C ¹H-NMR spectrum (300 MHz): (DMSO-D₆, HMDSO) δ: 8.55 (d, *J*=9.6 Hz, 1H), 8.07 (d, *J*=8.3 Hz, 1H), 7.80 (dd, *J*=7.3, 1.0 Hz, 1H), 7.76 (br s, 1H), 7.51 (dd, *J*=8.3, 7.3 Hz, 1H), 7.45 (dd, *J*=9.6, 2.7 Hz, 1H), 7.37 (br s, 1H), 7.11 (d, *J*=2.7 Hz, 1H), 3.28-3.38 (m, overlapped with water, 1H), 3.09 (s, 6H), 2.68 (d, *J*=7.2 Hz, 1H), 2.47 (d, *J*=4.4 Hz, 1H); ¹³C-NMR spectrum (100 MHz): (DMSO-D₆, HMDSO) δ: 171.8, 154.0, 141.2, 138.8, 137.3, 130.9, 130.0, 129.7, 125.7, 123.3, 111.3, 45.8, 41.2, 36.9; LCMS ESI⁺ (m/z): 320.3 [M+H]⁺, LCMS purity 100% |
| C-3511 | [1-(4-Hexylphenyl)sulfonylaziridin-2-yl]-pyrrolidin-1-yl-methanone |
| | Molecular formula: C₁₉H₂₈N₂O₃S; Molecular weight: 364.50; Melting point: oil |
| | ¹H-NMR spectrum (400 MHz): (CDCl₃, HMDSO) δ: 7.85 (d, *J*=8.3 Hz, 2H), 7.34 (d, *J*=8.3 Hz, 2H), 3.75 (dt, *J*=10.1*,* 6.7 Hz, 1H), 3.63 (dt, *J*=10.1*,* 7.0 Hz, 1H), 3.42-3.54 (m, 3H), 2.70 (d, *J*=4.2 Hz, 1H), 2.68 (t, *J*=7.9 Hz, 2H), 2.64 (d, *J*=6.9 Hz, 1H), 1.93-2.05 (m, 2H), 1.81-1.93 (m, 2H), 1.57-1.69 (m, 2H), 1.23-1.38 (m, 6H), 0.88 (t, *J*=7.0 Hz, 3H); ¹³C-NMR spectrum (100 MHz): (CDCl₃, HMDSO) δ: 163.4, 150.0, 134.4, 129.2, 128.3, 46.8, 46.5, 36.0, 35.8, 32.4, 31.6, 31.0, 28.9, 26.1, 24.2, 22.6, 14.1; LCMS ESI⁺ (m/z): 365.3 [M+H]⁺, LCMS purity 100% |
| C-3517 | [1-(4-Hexylphenyl)sulfonylaziridin-2-yl]-morpholino-methanone |
| | Molecular formula: C₁₉H₂₈N₂O₄S; Molecular weight: 380.18; Melting point: oil |
| | ¹H-NMR spectrum (400 MHz): (CDCl₃, HMDSO) δ: 7.85 (d, *J*=8.4 Hz, 2H), 7.36 (d, *J*=8.4 Hz, 2H), 3.75-3.86 (m, 2H), 3.57-3.75 (m, 5H), 3.55 (dd, *J*=6.8, 4.2 Hz, 1H), 3.46 (ddd, *J*=13.3, 7.7, 3.3 Hz, 1H), 2.71 (d, *J*=4.2 Hz, 1H), 2.69 (t, *J*=7.9 Hz, 2H), 2.67 (d, *J*=6.8 Hz, 1H), 1.57-1.68 (m, 2H), 1.23-1.39 (m, 6H), 0.88 (t, *J*=7.0 Hz, 3H); ¹³C-NMR spectrum (100 MHz): (CDCl₃, HMDSO) δ: 163.7, 150.3, 134.3, 129.3, 128.2, 66.7, 66.6, 42.8, 36.0, 35.3, 32.4, 31.6, 31.0, 28.9, 22.7, 14.1; LCMS ESI⁺ (m/z): 381.3 [M+H]⁺, LCMS purity 100% |
| C-3520 | [1-(4-hexylphenyl)sulfonylaziridin-2-yl]-(1-piperidyl)methanone |
| | Molecular formula: C₂₀H₃₀N₂O₃S; Molecular weight: 378.53; Melting point: oil |
| | ¹H-NMR spectrum (400 MHz): (CDCl₃, HMDSO) δ: 7.86 (d, *J*=8.4 Hz, 2H), 7.35 (d, *J*=8.4 Hz, 2H), 3.62-3.73 (m, 2H), 3.54 (dd, *J*=6.8, 4.2 Hz, 1H), 3.52-3.60 (m, 1H), 3.38-3.46 (m, 1H), 2.69 (t, *J*=7.3 Hz, 2H), 2.70 (d, *J*=4.2 Hz, 1H), 2.66 (d, *J*=6.8 Hz, 1H), 1.60-1.70 (m, 4H), 1.49-1.60 (m, 4H), 1.24-1.38 (m, 6H), 0.88 (t, *J*=7.0 Hz, 3H); ¹³C-NMR spectrum (100 MHz): (CDCl₃, HMDSO) δ: 163.3, 150.0, 134.6, 129.2, 128.2, 46.9, 43.6, 36.0, 35.7, 32.3, 31.6, 31.0, 28.9, 26.4, 25.4, 24.4, 22.6, 14.1; LCMS ESI⁺ (m/z): 379.3 [M+H]⁺, LCMS purity 100% |
| C-3532 | 1-(Benzenesulfonyl)aziridine-2-carboxamide |
| | Molecular formula: C₉H₁₀N₂O₃S; Molecular weight: 226.25; Melting point: 108-110 °C. ¹H-NMR spectrum (400 MHz): (CDCl₃, HMDSO) δ: 7.93-7.98 (m, 2H), 7.67-7.73 (m, 1H), 7.55-7.63 (m, 2H), 6.11 (s, 1H), 5.82 (s, 1H), 3.28 (dd, *J*=7.7, 4.2 Hz, 1H), 2.81 (d, *J*=7.7 Hz, 1H), 2.45 (d, *J*=4.2 Hz, 1H); ¹³C-NMR spectrum (100 MHz): (CDCl₃, HMDSO) δ: 168.3, 136.6, 134.4, 129.5, 128.2, 37.7, 33.2; LCMS ESI⁺ (m/z): 227.2 [M+H]⁺, LCMS purity 100.00% |
| C-3535 | Methyl (2S)-1-(p-tolylsulfonyl)aziridine-2-carboxylate |
| | Molecular formula: C₁₁H₁₃NO₄S; Molecular weight: 255.29. Melting point: oil. ¹H-NMR spectrum (400 MHz): (CDCl₃, HMDSO) δ: 7.84 (d, *J*=8.3 Hz, 2H), 7.36 (d, *J*=8.3 Hz, 2H), 3.74 (s, 3H), 3.34 (dd, *J*=7.1, 4.1 Hz, 1H), 2.76 (d, *J*=7.1 Hz, 1H), 2.56 (d, *J*=4.1 Hz, 1H), 2.45 (s, 3H). ¹³C-NMR spectrum (100 MHz (CDCl₃, HMDSO) δ: 167.2, 145.3, 134.0, 129.9, 128.2, 52.9, 35.7, 32.0, 21.7. LCMS ESI⁺ (m/z): 256.1 [M+H]⁺, LCMS purity 100% |
| C-3537 | Ethyl 1-(p-tolylsulfonyl)aziridine-2-carboxylate |
| | Molecular formula: C₁₂H₁₅NO₄S. Molecular weight: 269.32. Melting point: oil. ¹H-NMR spectrum (400 MHz): (CDCl₃, HMDSO) δ: 7.85 (d, *J*=8.3 Hz, 2H), 7.35 (d, *J*=8.3 Hz, 2H), 4.12-4.24 (m, 2H), 3.32 (dd, *J*=7.1, 4.1 Hz, 1H), 2.75 (d, *J*=7.1 Hz, 1H), 2.55 (d, *J*=4.1 Hz, 1H), 2.45 (s, 3H), 1.25 (t, *J*=7.1 Hz, 3H). ¹³C-NMR spectrum (100 MHz (CDCl₃, HMDSO) δ: 166.8, 145.2, 134.1, 130.0, 128.3, 62.1, 35.9, 32.0, 21.7, 14.0. LCMS ESI⁺ (m/z): 270.2 [M+H]⁺, LCMS purity 100% |
| C-3539 | Methyl (2R)-1-(p-tolylsulfonyl)aziridine-2-carboxylate |
| | Molecular formula: C₁₁H₁₃NO₄S; Molecular weight: 255.29. Melting point: oil. ¹H-NMR spectrum (400 MHz): (CDCl₃, HMDSO) δ: 7.84 (d, *J*=8.3 Hz, 2H), 7.36 (d, *J*=8.3 Hz, 2H), 3.74 (s, 3H), 3.34 (dd, *J*=7.1, 4.1 Hz, 1H), 2.76 (d, *J*=7.1 Hz, 1H), 2.56 (d, *J*=4.1 Hz, 1H), 2.45 (s, 3H). ¹³C-NMR spectrum (100 MHz (CDCl₃, HMDSO) δ: 167.2, 145.3, 134.0, 129.9, 128.2, 52.9, 35.7, 32.0, 21.7. LCMS ESI⁺ (m/z): 256.1 [M+H]⁺, LCMS purity 100%. ¹³C-NMR spectrum (100 MHz (CDCl₃, HMDSO) δ: 167.3, 145.3, 134.0, 130.0, 128.3, 53.0, 35.7, 32.1, 21.7. LCMS ESI⁺ (m/z): 256.1 [M+H]⁺, LCMS purity 97.75% |
| C-3546 | N,N-Diethyl-1-(4-hexylphenyl)sulfonyl-aziridine-2-carboxamide |
| | Molecular formula: C₁₉H₃₀N₂O₃S; Molecular weight: 366.52; Melting point: oil |
| | ¹H-NMR spectrum (400 MHz): (CDCl₃, HMDSO) δ: 7.86 (d, *J*=8.4 Hz, 2H), 7.34 (d, *J*=8.4 Hz, 2H), 3.56 (dq, *J*=15.0, 7.2 Hz, 1H), 3.54 (dd, *J*=6.8, 4.2 Hz, 1H), 3.49 (dq, *J*=15.0, 7.2 Hz, 1H), 3.32-3.44 (m, 2H), 2.69 (d, *J*=4.2 Hz, 1H), 2.69 (t, *J*=7.9 Hz, 2H), 2.65 (d, *J*=6.9 Hz, 1H), 1.56-1.67 (m, 2H), 1.26-1.38 (m, 6H), 1.26 (t, *J*=7.2 Hz, 3H), 1.13 (t, *J*=7.2 Hz, 3H), 0.89 (t, *J*=7.0 Hz, 3H); ¹³C-NMR spectrum (100 MHz): (CDCl₃, HMDSO) δ: 164.3, 150.0, 134.5, 129.2, 128.2, 42.1, 41.1, 36.0, 34.9, 32.5, 31.6, 31.0, 28.8, 22.6, 14.7, 14.1, 12.8; LCMS ESI⁺ (m/z): 367.3 [M+H]⁺, LCMS purity 100% |
| C-3548 | Methyl (2S)-1-[[6-(dimethylamino)-1-naphthyl]sulfonyl]aziridine-2-carboxylate |
| | Molecular formula: C₁₆H₁₈N₂O₄S; Molecular weight: 334.39; Melting point: 118-120°C |
| | ¹H-NMR spectrum (400 MHz): (CDCl₃, HMDSO) δ: 8.73 (d, *J*=9.6 Hz, 1H), 7.91 (d, *J*=8.3 Hz, 1H), 7.87 (dd, *J*=7.3, 1.2 Hz, 1H), 7.39 (dd, *J*=8.3, 7.4 Hz, 1H), 7.33 (dd, *J*=9.6, 2.7 Hz, 1H), 6.93 (d, *J*=2.7 Hz, 1H), 3.70 (s, 3H), 3.44 (dd, *J*=7.1, 4.1 Hz, 1H), ), 3.08 (s, 6H), 2.83 (d, *J*=7.1 Hz, 1H), 2.55 (d, *J*=4.2 Hz, 1H); ¹³C-NMR spectrum (100 MHz): (CDCl₃, HMDSO) δ: 167.3, 148.9, 136.2, 133.8, 132.0, 126.2, 125.6, 124.1, 121.5, 118.2, 106.2, 52.8, 40.4, 35.7, 32.3. LCMS ESI⁺ (m/z): 335.2 [M+H]⁺, LCMS purity 100% |
| C-3559 | Methyl 1-(3-pyridylsulfonyl)aziridine-2-carboxylate |
| | Molecular formula: C₉H₁₀N₂O₄S; Molecular weight: 242.25; Melting point: oil |
| | ¹H-NMR spectrum (400 MHz): (CDCl₃, HMDSO) δ: 9.17 (dd, *J*=2.3, 0.7 Hz, 1H), 8.90 (dd, *J*=4.9, 1.6 Hz, 1H), 8.28 (ddd, *J*=8.1, 2.3, 1.7 Hz, 1H), 7.53 (ddd, *J*=8.1, 4.9, 0.7 Hz, 1H), 3.76 (s, 3H), 3.45 (dd, *J=*7.1*,* 4.2 Hz, 1H), 2.87 (d, J=7.1 Hz, 1H), 2.65 (d, *J*=4.2 Hz, 1H); ¹³C-NMR spectrum (100 MHz): (CDCl₃, HMDSO) δ: 166.88, 154.7, 148.8, 135.9, 134.2, 124.1, 53.1, 36.0, 32.4; LCMS ESI⁺ (m/z): 243.1 [M+H]⁺, LCMS purity 92.21% |
| C-3562 | 1-(3-pyridylsulfonyl)aziridine-2-carboxamide |
| | Molecular formula: C₈H₉N₃O₃S; Molecular weight: 227.24; Melting point: 147-149°C |
| | ¹H-NMR spectrum (400 MHz): (DMSO-D₆, HMDSO) δ: 9.11 (dd, *J*=2.3, 0.7 Hz, 1H), 8.96 (dd, *J*=4.9, 1.6 Hz, 1H), 8.38 (ddd, *J*=8.1, 2.3, 1.6 Hz, 1H), 7.87 (s, 1H), 7.53 (ddd, *J*=8.1, 4.9, 0.7 Hz, 1H), 7.46 (s, 1H), 3.34-3.38 (m, 1H, overlapped with H₂O), 2.77 (d, *J*=7.2 Hz, 1H), 2.56 (d, *J=*4.5 Hz, 1H); ¹³C-NMR spectrum (100 MHz): (DMSO-D₆, HMDSO) δ: 166.69, 155.3, 148.5, 136.4, 134.1, 125.1, 37.6, 32.0; LCMS ESI⁺ (m/z): 228.1 [M+H]⁺, LCMS purity 100% |
| C-3570 | Methyl (2R)-1-[[6-(dimethylamino)-1-naphthyl]sulfonyl]aziridine-2-carboxylate |
| | Molecular formula: C₁₆H₁₈N₂O₄S; Molecular weight: 334.39; Melting point: 117-119°C ¹H-NMR spectrum (400 MHz): (CDCl₃, HMDSO) δ: 8.73 (d, *J*=9.5 Hz, 1H), 7.91 (d, *J*=8.3 Hz, 1H), 7.87 (dd, *J*=7.3, 1.2 Hz, 1H), 7.39 (dd, *J*=8.3, 7.4 Hz, 1H), 7.33 (dd, *J*=9.5, 2.7 Hz, 1H), 6.93 (d, *J*=2.7 Hz, 1H), 3.70 (s, 3H), 3.44 (dd, *J*=7.1, 4.1 Hz, 1H), ), 3.08 (s, 6H), 2.83 (d, *J*=7.1 Hz, 1H), 2.55 (d, *J*=4.2 Hz, 1H); ¹³C-NMR spectrum (100 MHz): (CDCl₃, HMDSO) δ: 167.4, 148.9, 136.3, 133.8, 132.0, 126.2, 125.6, 124.2, 121.6, 118.2, 106.3, 52.8, 40.4, 35.7, 32.4; LCMS ESI⁺ (m/z): 335.3 [M+H]⁺, LCMS purity 100% |
| C-3576 | Methyl 1-(4-fluorophenyl)sulfonylaziridine-2-carboxylate |
| | Molecular formula: C₁₀H₁₀FNO₄S; Molecular weight: 259.25; Melting point: oil |
| | ¹H-NMR spectrum (400 MHz): (CDCl₃, HMDSO) δ: 7.96-8.02 (m, 2H), 7.21-7.27 (m, 2H), 3.74 (s, 3H), 3.37 (dd, *J*=7.1, 4.1 Hz, 1H), 2.79 (d, *J*=7.1 Hz, 1H), 2.59 (d, *J*=4.1 Hz, 1H); ¹³C-NMR spectrum (100 MHz): (CDCl₃, HMDSO) δ: 167.1, 166.1 (d, *J*=257.1 Hz, 1H), 133.2 (d, *J=3.1* Hz, 1H), 131.1 (d, *J*=9.9 Hz, 1H), 116.7 (d, *J*=22.9 Hz, 1H), 53.0, 35.9, 32.2; LCMS ESI⁺ (m/z): 260.2 [M+H]⁺, LCMS purity 100% |
| C-3591 | 1-(4-tert-Butylphenyl)sulfonylaziridine-2-carboxamide |
| | Molecular formula: C₁₃H₁₈N₂O₃S. Molecular weight: 282.36. Melting point: 151-153°C. ¹H-NMR spectrum (400 MHz): (CDCl₃, HMDSO) δ: 7.86 (d, *J*=8.8 Hz, 2H), 7.59 (d, *J*=8.8 Hz, 2H), 6.07 (br s, 1H), 5.42 (br s, 1H), 3.24 (dd, *J*=7.7, 4.2 Hz, 1H), 2.81 (d, *J*=7.7 Hz, 1H), 2.44 (d, *J=*4.2 Hz, 1H), 1.36 (s, 9H). ¹³C-NMR spectrum (100 MHz (CDCl₃, HMDSO) δ: 168.4, 158.5, 133.2, 128.1, 126.5, 37.6, 35.4, 33.2, 31.4. LCMS ESI⁺ (m/z): 283.3 [M+H]⁺, LCMS purity 100% |
| C-3593 | 1-(4-tert-Butylphenyl)sulfonyl-N-methylaziridine-2-carboxamide |
| | Molecular formula: C₁₄H₂₀N₂O₃S. Molecular weight: 296.38. Melting point: 146-148°C. ¹H-NMR spectrum (400 MHz): (CDCl₃, HMDSO) δ: 7.85 (d, *J*=8.7 Hz, 2H), 7.59 (d, *J*=8.7 Hz, 2H), 6.16 (br s, 1H), 3.28 (dd, *J*=7.7, 4.2 Hz, 1H), 2.76 (d, *J*=4.9 Hz, 3H), 2.73 (d, *J*=7.7 Hz, 1H), 2.36 (d, *J*=4.2 Hz, 1H), 1.36 (s, 9H). ¹³C-NMR spectrum (100 MHz (CDCl₃, HMDSO) δ: 166.3, 158.5, 133.1, 128.2, 126.5, 37.9, 35.4, 33.5, 31.0, 26.0. LCMS ESI⁺ (m/z): 297.3 [M+H]⁺ , LCMS purity 100% |
| C-3594 | 1-(4-tert-Butylphenyl)sulfonyl-N,N-dimethyl-aziridine-2-carboxamide |
| | Molecular formula: C₁₅H₂₂N₂O₃S. Molecular weight: 310.41. Melting point: 104-106°C. ¹H-NMR spectrum (400 MHz): (CDCl₃, HMDSO) δ: 7.88 (d, *J*=8.6 Hz, 2H), 7.56 (d, *J*=8.6 Hz, 2H), 3.23 (s, 3H), 3.60 (dd, *J*=6.8, 4.2 Hz, 1H), 3.23 (s, 3H), 2.98 (s, 3H), 2.69 (d, *J*=6.8 Hz, 1H), 2.66 (d, *J*=4.2 Hz, 1H), 1.35 (s, 9H). ¹³C-NMR spectrum (100 MHz (CDCl₃, HMDSO) δ: 165.0, 159.1, 134.2, 128.0, 126.3, 37.3, 36.0, 35.3, 35.2, 31.1. LCMS ESI⁺ (m/z): 311.3 [M+H]⁺ , LCMS purity 100% |
| C-3612 | Lithium 1-tosylaziridine-2-carboxylate |
| | Molecular formula: C₁₀H₁₀LiNO₄S; Molecular weight: 247.20; Melting point: 222-224 °C (decomp.); ¹H-NMR spectrum (400 MHz): (D₂O, HMDSO) d: 7.84 (d, *J*=8.3 Hz, 2H), 7.49 (d, *J*=8.3 Hz, 2H), 3.10 (dd, *J*=7.1, 4.1 Hz, 1H), 2.74 (d, *J*=7.1 Hz, 1H), 2.51 (d, *J*=4.1 Hz, 1H), 2.44 (s, 3H); ¹³C-NMR spectrum (100 MHz): (D₂O, HMDSO) d: 173.2, 146.7, 131.6, 130.2, 127.9, 39.0, 32.3, 20.8.LCMS ESI⁻ (m/z): 240.2 [M-Li]⁻, LCMS purity 95.83% |

### Example 2. Inhibition of PDI A3

The inhibitory effects compounds of invention on activity of PDIA3 was assess based on the insulin turbidimetric assay. Enzymatic activity of PDIA3 was confirmed by measuring the turbidity increase at 650 nm due to insulin reduction. The assay mixture was prepared by addition 10ug/ml PDI A3 (*E.coli* recombinant protein; Mybiosource), 0.1 mM phosphate buffer (pH7.6), 1mM EDTA, 0.087 mM DTT and with or without tested compound and was incubated for 60 min, at 37°C. Reaction was started by addition of insulin and DTT. Final concentration of insulin and DTT in assay mixture were 0.15 mM and 0.174 mM, respectively. Turbidity was detected at 650 nm against reference samples without PDIs. The measurements were performed at 650 nm using 120-s recordings.. The table 2 shows the results of the PDI A3 inhibition by disclosed compounds.

**TABLE 2. Inhibition of PDI A3**

| Compound Number | Inhibition of PDI A3 (EC 50, µM) | Compound Number | Inhibition of PDI A3 (EC 50, µM) | Compound Number | Inhibition of PDI A3 (EC 50, µM) |
|---|---|---|---|---|---|
| C-3376 | 2,8 | C-3314 | 24.0 | C-3355 | 61.0 |
| C-3399 | 4.0 | C-3364 | 24.0 | C-3294 | 68.0 |
| C-3390 | 4.0 | C-3403 | 24.0 | C-3320 | 69.0 |
| C-3375 | 4,3 | C-3393 | 27.0 | C-3570 | 68.4 |
| C-3377 | 4,3 | C-3161 | 27.0 | C-3291 | 77.0 |
| C-3548 | 5,3 | C-3316 | 28.0 | C-3295 | 78.0 |
| C-3373 | 5,4 | C-3576 | 28.0 | C-3326 | 84.0 |
| C-3400 | 6.7 | C-3290 | 29.0 | C-3336 | 89.0 |
| C-3520 | 6,7 | C-3591 | 29.0 | C-3365 | 90.0 |
| C-3535 | 6.8 | C-3537 | 30.0 | C-3311 | 90.0 |
| C-3371 | 7.5 | C-3212 | 32.0 | C-3263 | 98.0 |
| C-3391 | 7.6 | C-3594 | 32.0 | C-3511 | 102.0 |
| C-3397 | 8.5 | C-3332 | 35.0 | C-3292 | 114.0 |
| C-3532 | 9,3 | C-3380 | 37.0 | C-3287 | 122.0 |
| C-3383 | 10.1 | C-3327 | 37.0 | C-3342 | 123.0 |
| C-3427 | 10.5 | C-3366 | 37.0 | C-3459 | 130.0 |
| C-3398 | 10.5 | C-3308 | 40.0 | C-3319 | 145.0 |
| C-3385 | 11.1 | C-3374 | 40.0 | C-3353 | 143.0 |
| C-3384 | 11.7 | C-3272 | 43.0 | C-3304 | 190.0 |
| C-3343 | 14.0 | C-3216 | 48.0 | C-3357 | >200 |
| C-3517 | 14.7 | C-3257 | 48.0 | C-3305 | >200 |
| C-3369 | 18.0 | C-3299 | 48.0 | C-3256 | >200 |
| C-3559 | 18.6 | C-3350 | 48.0 | C-3270 | >200 |
| C-3362 | 19.4 | C-3271 | 52.5 | C-3281 | >200 |
| C-3220 | 22.0 | C-3251 | 52.5 | C-3297 | >200 |
| C-3389 | 23.3 | C-3562 | 56.0 | C-3303 | >200 |
| C-3368 | 24.0 | | | | |

### Example 3. Evaluation of anti-aggregatory effect of PDI A3 inhibitors in vitro and their anti-thrombotic effects in vivo

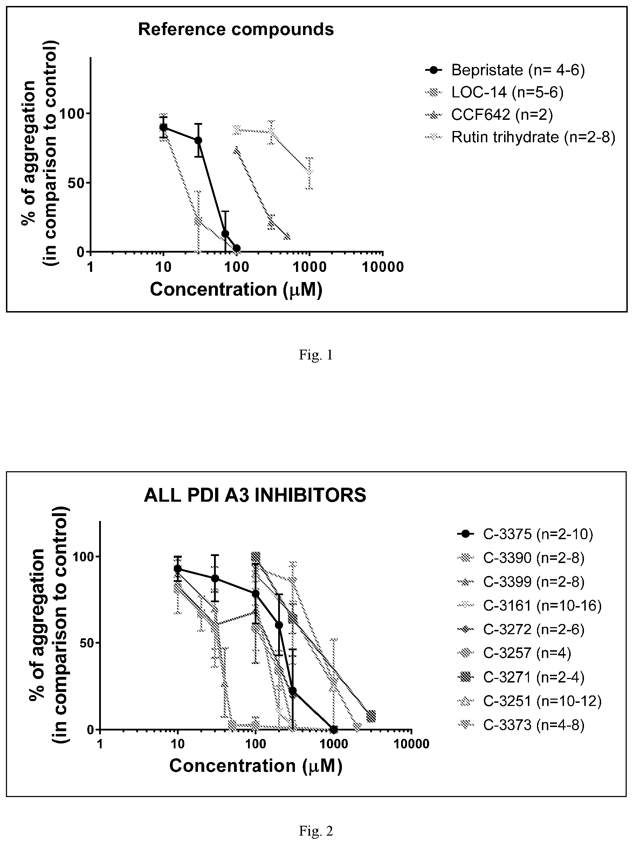

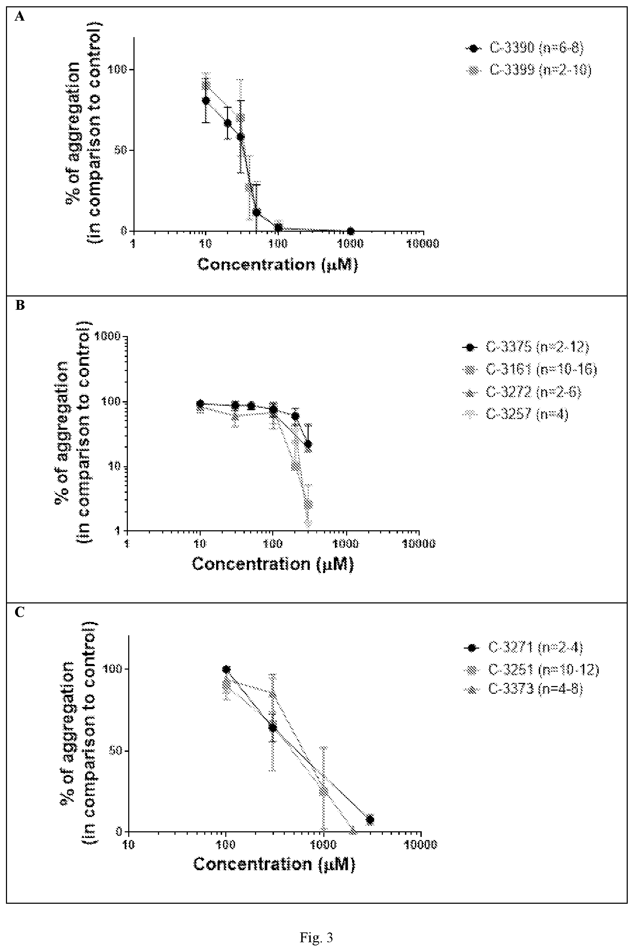

Anti-platelet activity of compounds of invention was assessed using light transmission aggregometry assay of human platelet-rich plasma (PRP). Venous blood was obtained from male volunteers at the University Hospital Blood Bank Centre. Volunteer donors had not taken any medicines for the preceding two weeks. Blood was collected into vials containing sodium citrate (3.2%, 9: 1v/v) as an anti-coagulant agent. To obtain platelet rich plasma (PRP), blood was centrifuged at 260 × g for 15 min. The platelet pure plasma fraction (used as a blank sample) was obtained by centrifugation of the remaining blood for 10 min at 2600 x g. Aggregation of blood platelets was assessed in PRP with a dual channel Chronolog aggregometer (CHRONO-LOG) using a method previously described by Born³⁵. PRP (500 µl) was equilibrated for 2 min at 37° C with a continuous stirring at 800 rpm and then stimulated with collagen to cause aggregation. At the beginning of each experiment, concentrations of collagen that induced sub-maximum aggregation response were determined. These concentrations were in the range of 1-5 mg/ml, respectively. All the tested compounds were added 2 min prior to stimulation of platelets with collagen. Transmittance was read 6 min after stimulation of platelets with an agonist. Figure 1 present shows inhibition of aggregation of human platelets by reference PDI inhibitors; while Fig. 2, 3 show inhibition of aggregation of human platelets by PDI A3 inhibitors of the invention.

As shown on fig. 1, inhibition of platelet aggregation by reference PDI inhibitors in human PRP (Bepristat, LOC-14, CCF642 and rutin trihydrate) was tested. Data represent the means ± SD of at least two independent experiments; n = 2-8 replicates in each experiment.

Fig. 2 shows inhibition of platelet aggregation by 7 tested compounds among PDI A3 inhibitors according to invention. Experiments were performed in human PRP, data are shown in comparison to control; data represent the means ± SD of at least two independent experiments; n = 2-10 replicates in each experiment. The most promising compounds seem to be C-3390 and C-3399: as these two compounds have the lowest EC50 and their antiplatelet effects is shown on fig. 3A. The other compounds have a lower potency, but all of them have a dose-dependent inhibitory effect on platelet aggregation in human platelet rich plasma as shown in fig. 3B and 3C.

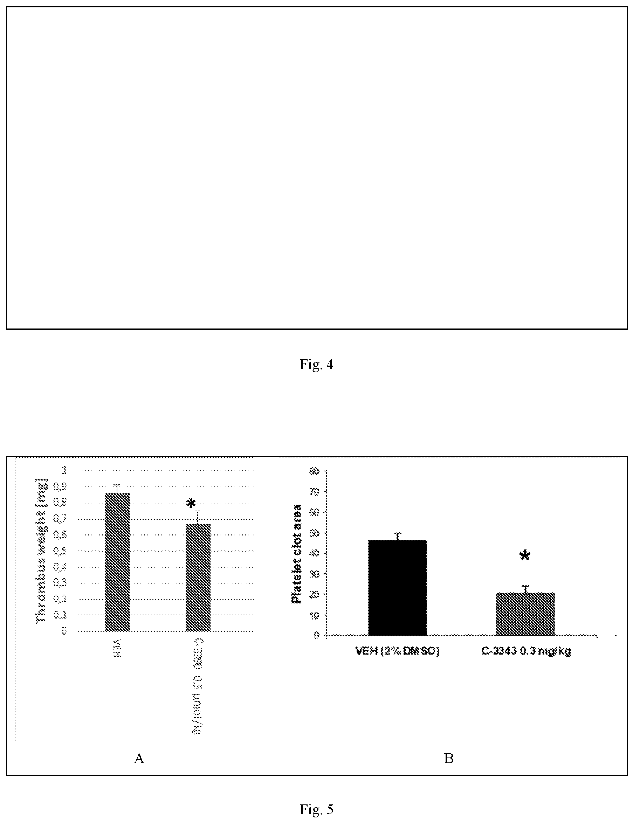

As shown of fig. 4, there was a significant correlation between inhibiting PDI A3 (EC50) of selected PDI A3 inhibitors and theirs inhibitory effect on platelet aggregation (in 100 µM concentration). Experiments were performed in human PRP, data represent the means of at least two independent experiments; n = 2-10 replicates in each experiment. These results support strongly the notion that inhibitory effects on platelet aggregation of compounds of invention is related to the inhibition of PDI A3.

To confirm antithrombotic activity of compounds of invention, inhibitors of PDIA3, pharmacological activity of selected compounds was tested *in vivo* in the rat or mice model of arterial thrombosis . Wistar rats were anaesthetized with pentobarbital (40 mg/kg, i.p.) and placed in a supine position on a heated (37 °C) operating table. Arterial thrombosis was induced by electrical stimulation of the right common carotid artery, as previously described (Kramkowski et al., 2012). Briefly, the anode, a stainless steel L-shaped wire, was inserted under the artery and connected to a constant current generator. The cathode was attached subcutaneously to the hind limb. The artery was stimulated (1 mA) for 10 min. Fifty-five minutes after the beginning of stimulation, the segment of the common carotid artery containing the formed thrombus was dissected and opened lengthwise, and the thrombus was completely removed and air-dried at room temperature for 24 h. Thrombus was then weighed in a blinded manner. Antithrombotic effects of compounds of invention in mice was analyzed using intravital fluorescence confocal microscopy as described previously (Hayashi T et al., 2008). GFP mice were anesthetized with pentobarbital (50 mg/kg, i.p.) a midline laparotomy incision was made, and then the mesentery of the ileum was pulled out of the abdomen and draped over a plastic mound. The mesentery was continuously perfused with 37°C-warmed saline to prevent the vessels from drying. Mesenteric vessels were identified and endothelial injury was induced by a 514-nm argon-ion laser. For visualizing the surface-exposed platelet phospholipids in a thrombus, Alexa Fluor 568-labeled annexin V (ANX; 2-µg/g mouse body weight) was administered into the right femoral vein 14 minutes before laser injury. Changes in fluorescence intensity were measured as described previously (Hayashi T et al., 2008, Kramkowski et al., 2002)

Fig 5 shows anti-thrombotic effects of C3990 and C-3343 two chosen compounds of invention in *in vivo* models of arterial thrombosis.

### Example 4. The in vitro antiproliferative effect of PDI A3 inhibitors towards panel of cancer cells (48-hour exposition)

Anticancer activity of compounds of invention, PDI A3-inhibitors has been tested *in vitro* in classical antiproliferative assay in various cancer cells lines. Monolayer tumor cell lines MDA-MB-231 (human mammary breast adenocarcinoma), MCF-7 (human breast adenocarcinoma, estrogen-positive), HT-1080 (human fibrosarcoma) and Caco-2 (human colon adenocarcinoma) were cultured in standard medium DMEM (Dulbecco's modified Eagle's medium) ("Sigma") supplemented with 10% foetal bovine serum ("Sigma"). About 2000-4000 cells per well (depending on line nature) were placed in 96-well plates and after 24h compounds were added to the wells. Untreated cells were used as a control. The plates were incubated for 48 h, 37°C, 5% CO₂. The number of surviving cells was determined using 3-(4,5-dimethylthiazol-2-yl)-2,5-diphenyltetrazolinium bromide (MTT). MTT-test: after incubating culture medium was removed and 200 µL fresh medium with 20 µL MTT (2mg/mL in HBSS) was added in each well of the plate. After incubation (3 hr., 37°C, 5% CO₂), the medium with MTT was removed and 200 µL DMSO were added at once to each sample. The samples were tested at 540 nm on Thermo Scientific Multiszkan EX microplate photometer. The half-maximal inhibitory concentration (IC₅₀) of each compound was calculated using Graph Pad Prism^{®} 3.0. The results are presented in Table 5.

**TABLE 5. The in vitro antiproliferative effect of PDI A3 inhibitors towards panel of cancer cells (48-hour exposition)**

| Comp. | Cytotoxicity, IC50, µM | | | |
|---|---|---|---|---|
| | HT-1080 | CaCo-2 | MDA-MB-231 | MCF-7 |
| C-3212 | 56±9 | 45±4 | 46±8 | 54±4 |
| C-3216 | 15±2 | 40±5 | 22±1 | 16±0.2 |
| C-3218 | 200±3 | 200±1 | 64±5 | 137±14 |
| C-3220 | 5.9±1.1 | 56±4 | 45±9 | 35±6 |
| C-3256 | 215±5 | 205±29 | 8.9±1.6 | 2.8±3.2 |
| C-3262 | 3.8±0.3 | 13.5±0.8 | 6.8±0.8 | 7.2±0.8 |
| C-3263 | 7.6±0.2 | 29±0.8 | 9.1±0.5 | 17±0.2 |
| C-3270 | 21±3 | 87±4 | 52±5 | 33±4 |
| C-3271 | 49±6 | 138±11 | 13±2 | 54±4 |
| C-3272 | 4.5±0.9 | 57±6 | 8.4±0.9 | 26±0.4 |
| C-3273 | 31±0.9 | 56±12 | 35±3 | 92±2 |
| C-3281 | 164±3.2 | 125±6.4 | 15±1 | 118±5 |
| C-3287 | 92±9.2 | 144±6 | 12±2 | 123±17 |
| C-3288 | 70±2.9 | 118±3 | 10±1 | 113±4 |
| C-3290 | 9.5±1.8 | 6.9±0.2 | 7.8±1.6 | 29±4 |
| C-3291 | 140±3 | 140±5 | 32±4 | 34±1 |
| C-3292 | 34±3.4 | 14.4±1.4 | 16±1 | 25±1 |
| C-3294 | 88.5±11 | 8.1±0.7 | 40±6 | 49±6 |
| C-3295 | 74.1±2.5 | 12±1.1 | 17±1 | 32±2 |
| C-3296 | 210±15 | 37±1.5 | 30±1 | 32±1 |
| C-3297 | 154±11 | 15.4±0.7 | 5.6±0.6 | 7.5±0.5 |
| C-3299 | 56±3 | 53±7 | 18±2 | 41±2 |
| C-3303 | 70±2 | 27±2 | 102±9 | 170±35 |
| C-3304 | 17±3 | 10±1 | 13±1 | 49±1 |
| C-3305 | 190±3 | 21±1 | 12±1 | 25±1 |
| C-3308 | 120±6 | 100±5 | >500 | 485±32 |
| C-3311 | 5.1±0.2 | 7.2±0.2 | 33±4 | 124±5 |
| C-3314 | 4.5±1 | 14±0.8 | 104±3 | 206±21 |
| C-3316 | 76±3 | 81±4 | 78±12 | 137±18 |
| C-3319 | 44±4 | 31±6 | 25±5 | 28±8 |
| C-3320 | 180±8 | 78±19 | 380±15 | >500 |
| C-3324 | 510±3 | 27±3 | 102±12 | 156±9 |
| C-3326 | 100±5 | 170±20 | 169±20 | 349±13 |
| C-3327 | 130±10 | 150±10 | 161±2 | 168±23 |
| C-3329 | 33±4 | 19±2 | 6.5±0.5 | 8.2±0.8 |
| C-3332 | 140±20 | 5.9±0.3 | 44±7 | 28±3 |
| C-3336 | 4.6±0.4 | 0.5±0.1 | 7.6±1.9 | 16±3 |
| C-3342 | 6.7±2 | 6±0.5 | 17±2 | 33±2 |
| C-3343 | 6.5±0.6 | 68±4 | 26±8 | 46±5 |
| C-3346 | 169±11 | 17±2 | >200 | 458±6 |
| C-3350 | 16±5 | 49±5 | 136±6 | 80±8 |
| C-3353 | 31±1 | >500 | 92±8 | 53±9 |
| C-3355 | 20±1 | 39±1 | 247±14 | 276±9 |
| C-3357 | 30±1 | 61±3 | 25±6 | 295±9 |
| C-3362 | 32±5 | 25±6 | 19.1±1 | 40±9 |
| C-3364 | 29±4 | 11±3 | 21±0.3 | 22±2 |
| C-3365 | 28±4 | 21±4 | 4.6±0.1 | 7.3±0.5 |
| C-3366 | 11±2 | 11±2 | 20±0.2 | 9.1±0.6 |
| C-3368 | 28±1 | 28±5 | 26±3 | 16±4 |
| C-3369 | 10±2 | 22±1 | 22±2 | 21±1 |
| C-3371 | 15±3 | 25±1 | 36±7 | 11±1 |
| C-3373 | 4.8±0.8 | 43±6 | 19±1 | 19±2 |
| C-3374 | 4.6±0.3 | 25±5 | 6.7±0.2 | 4.9±0.6 |
| C-3375 | 22±4 | 55±5 | 75±4 | 12±2 |
| C-3376 | 1.3±0.1 | 11±1 | 2.3±0.2 | 27±2 |
| C-3377 | 6.1±0.3 | 16±1 | 7.8±1.4 | 4.8±0.1 |
| C-3380 | 28±4 | 157±25 | 32±7 | 44±10 |
| C-3383 | 12±1 | 50±7 | 7.3±0.5 | 80±6 |
| C-3384 | 14±3 | 56±6 | 5.5±0.6 | 40±5 |
| C-3385 | 8.0±0.7 | 20±2 | 7.0±0.7 | 16±3 |
| C-3389 | 36±3 | 49±2 | 32±3 | 47±6 |
| C-3390 | 7.9±0.9 | 11±2 | 18±0.1 | 20±0.3 |
| C-3391 | 7.8±1.0 | 38±4 | 20±0.3 | 22±2 |
| C-3393 | 0.46±0.04 | 35±1 | 32±4 | 29±3 |
| C-3397 | 9.3±1.7 | 36±5 | 38±2 | 62±4 |
| C-3398 | 7.4±0.8 | 34±6 | 17±3 | 23±3 |
| C-3399 | 7.9±0.7 | 55±7 | 17±1 | 41±6 |
| C-3400 | 11±1 | 24±2 | 24±5 | 33±1 |
| C-3402 | 4.6±0.1 | 14±2 | 39±5 | 43±8 |
| C-3403 | 6,7±0,9 | 38±5 | 16±1 | 11±3 |
| C-3427 | 5.9±0.5 | 20±5 | 9.3±1 | 13±2 |
| C-3459 | >500 | n.e. | n.e. | n.e. |
| C-3511 | n.t. | 61±4 | 5.4±0.3 | 28±3 |
| C-3517 | n.t. | 74±4 | 12±1 | 18±1 |
| C-3520 | n.t. | 45±6 | 16±2 | 30±1 |
| C-3532 | 74±1 | 181±33 | 27±2 | 103±2 |
| C-3535 | 84±3 | 35±7 | 107±11 | 81±0 |
| C-3537 | 17±1 | 27±2 | 28±3 | 35±1 |
| C-3539 | 20±1 | 23±1 | 40±5 | 113±6 |
| C-3546 | 0.13±0.02 | 73±10 | 14±1 | 32±7 |
| C-3548 | 9.2±1.0 | 45±13 | 57±9 | 37±3 |
| C-3559 | 372±23 | 260±55 | 128±8 | 154±25 |
| C-3562 | 31±2 | 36±6 | 24±2 | 57±5 |
| C-3570 | 12±1 | 13±1 | 14±3 | 29±2 |
| C-3591 | 9.9±0.9 | 27±4 | 12±3 | 16±2 |
| C-3593 | 16±1 | 25±4 | 20±1 | 9±2 |
| C-3594 | 15±1 | 7.2±0.6 | 6.1±2.4 | 10±2 |

### Example 5. Antiproliferative effect of PDIA3-inhibitors in vitro in hypoxic conditions and in cancer cells stimulated with estrogen.

Anticancer activity of selected compounds of invention, PDI A3-inhibitors has been also tested *in vitro* in antiproliferative assay in normoxic and hypoxic conditions as well in estrogen-stimulated cancer cells. In the experiment, cells were seeded on 96-well plates (Sarstedt, Germany) in appropriate culture medium at a density of 10⁵ cells/mL 24h before adding the tested compounds. Cells were treated with each compound in four concentrations in the range 0,1-100 µg/mL. Cisplatin (Ebewe, Austria) in the range 0,01 -10 µg/mL was used as a reference drug. Dimethyl sulfoxide (DMSO), used as a stock solution solvent, was tested for antiproliferative activity and it did not affect cell proliferation at 0.1% (v/v) - a highest concentration used in compound solutions. After 72 h of compound treatment at 37 °C, 5 % CO₂ humid atmosphere and in wo different oxygen level conditions: 21% - normal and <1% - hypoxia. In some experiments the MDA-MB-231 and MCF-7 cells were seeded with or without 200nM estradiol and after 24 h the tested compounds were added. A previously described sulforhodamine B antiproliferative assay was used with minor modifications (Skehan P et al., 1990). Briefly, cells were fixed with 50 µL/well of 50% (w/v) trichloroacetic acid (Avantor Performance Materials, Gliwice, Poland). After 1 h incubation, plates were washed several times with tap water and 50 µL of 0.4% (w/v) solution of sulforhodamine B (Sigma-Aldrich, Germany) in 1% (v/v) acetic acid (Avantor Performance Materials, Gliwice, Poland) was added to each well. After 30 min incubation at room temperature, unbound dye was washed out with 1% (v/v) acetic acid, whereas bound dye was solubilized with 10 mM unbuffered TRIS (Avantor Performance Materials, Gliwice, Poland) solution. The entire procedure was performed using a BioTek EL-406 washing station (BioTek Instruments, USA). After additional 30 min, absorbance was read using a Biotek Hybrid H4 reader (BioTek Instruments, USA) at 540 nm wavelength. MTT assay was used alternatively for HL-60 cell line and in the experiment in which estrogens are added (specified in the table legend). Absorbance was measured using a Biotek Hybrid H4 reader at 570 nm wavelength for MTT assay.

Compounds at each concentration were tested in triplicate in a single experiment and each experiment was repeated at least three times independently. Results of *in vitro* antiproliferative effects are shown in Table 3 and Table 4. Results are presented as mean IC₅₀ ± SD.

**TABLE 3. Antiproliferative effect of PDI A3 inhibitors in normal (21% of oxygen) and hypoxia (<1% of oxygen) conditions against human cancer cell lines (72 hours exposition).**

| Compd | Cell line/condition IC50 [µg/ml] | | | | | |
|---|---|---|---|---|---|---|
| | **MCF-7** | | **MDA-MB-231** | | **Caco-2** | **HT-29** |
| | normal | hypoxia | normal | hypoxia | normal | normal |
| C-3216 | 3.7 ± 0.4 | 3.6 ± 0.3 | 5.0 ± 1.4 | 6.2 ± 3.7 | 4.5 ± 0.8 | 5.6 ± 0.9 |
| CCF642 | 0.7 ± 0.5 | 0.6 ± 0.6 | 0.3 ± 0.1 | 0.3 ± 0.2 | 0.4 ± 0.1 | 0.4 ± 0.05 |
| LOC14 | 4.4 ± 0.9 | 3.9 ± 0.3 | 2.8 ± 0.4 | 2.6 ± 0.5 | 9.1 ± 3.4 | 12.6 ± 8.4 |
| Cisplatin | 1.8 ± 0.3 | 1.3 ± 0.5 | 3.2 ± 0.5 | 3.1 ± 0.9 | 2.3 ± 0.5 | 2.82 ± 0.6 |

**TABLE 4. The antiproliferative effect of PDI A3 inhibitors towards human breast cancer cell lines pretreated or not with estradiol and for comparison towards human colon cancer and leukemia cell line (72 hours exposition).**

| Cmpd | Cell line/condition IC50 [µg/ml] | | | | | | *Selectivity index (SI): IC50 of MCF-10A*/ *IC50 of MCF-7* |
|---|---|---|---|---|---|---|---|
| | **MDA-MB-231** | | **MCF-7** | | **HT-29** | **HL-60** | |
| | **normal** | **+ estradiol** | **normal** | **+ estradiol** | **normal** | **normal** | |
| C-3373 | 3.2 ± 0.1 | 3.2 ± 0.3 | 3.3 ± 0.1 | 2.6 ± 0.5 | 33.4 ± 1.1 | 3.1 ± 0.1 | *1.20* |
| C-3375 | 3.8 ± 0.1 | 7.0 ± 1.1* | 3.5 ± 0.4 | 3.4 ± 0.3 | 27.8 ± 7.5 | 3.3 ± 0.2 | *6.90* |
| C-3390 | 3.2 ± 0.2 | 9.4 ± 3.5 | 3.2 ± 0.3 | 3.4 ± 0.3 | 33.6 ± 2.9 | 14.4 ± 0.7 | *5.10* |
| C-3399 | 2.9 ± 0.3 | 6.4 ± 1.9 | 3.7 ± 0.3 | 4.3 ± 0.3 | 4.3 ± 0.2 | 2.4 ± 0.3 | *1.6* |

### Statistical analysis: Unpaired t test. *p<0.05 as compared to appropriate cells not pretreated with estradiol. MTT assay was used for antiproliferative activity assessment.

In reference to Table 3 and Table 4, all new compounds tested in normal condition revealed antiproliferative activity comparable to LOC14. In reference to Table 3, the antiproliferative effect of C-3216 and reference inhibitors was comparable and normoxic and hypoxic conditions supporting the notion that antiproliferative effect of compounds of invention on human breast cancer cells MCF-7 and MDA-MB-231 is not dependent on oxygen access. Therefore, these compounds are effective in the hypoxic environment that occurs in the tumor. In reference to Table 4, it was established that the MDA-MB-231 line after treatment with estradiol (200 nM) shows slightly lower sensitivity to PDI A3 inhibitors: C-3375, C-3390, C-3399, a phenomenon that was not observed in MCF-7 breast cancer cell line.

### Example 6. Evaluation of effects of PDI A3 inhibitors on transendothelial cancer cells migration in vitro

To assess whether compounds of invention, PDI A3-inhibitors are also effective as inhibitors of cancer cell transmigration through endothelium the transmigration assay with MDA-MB-231/lung microvascular endothelium was used as described previously (Stojak et al., 2018). Cell migration was assayed in 24-well, 6.5-mm internal-diameter Transwell plates (8.0 µm pore size; BD Pharmingen). Human lung microvascular endothelial cells (hLMVECs) were seeded into 24-well plates (seeding density 5×104 cells/insert) on the upper side of the filter and left to grow to confluence. After confluent monolayer formation, hLMVECs were pre-treated with 10 ng/mL IL-1β for 6 h. Prior to use in transmigration assay, cancer cells were pre-incubated with various concentrations (3, 10, 30, 50, 100 µM) of tested inhibitors of PDIA3, C-3390, C-3399 for 30 min. Then, MDA-MB-231 cells (each 5×104 per well) were placed into upper chambers and tested PDI inhibitors at various concentrations (1, 10, 30, 50, 100 µM) were given. Lower chambers were filled with medium containing chemoattractant (20% FBS or 100 ng/mL SDF-1α). After 24 h of co-culture, hLMVEC monolayers and non-migrating cancer cells on the upper surface of the membrane were removed. Migrated cancer cells on the undersides of the Transwell membranes were detached and stained by Calcein-AM-Accutase solution for 60 minutes. The cell number was determined by measuring the fluorescence using plate reader. Experiments were performed in triplicates and repeated three times.

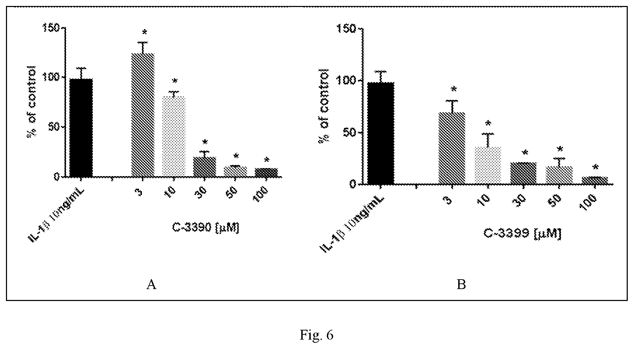

In reference to fig. 6a, compound of invention C-3390 inhibited MDA-MB-231 cell transmigration across IL-1β 10 ng/mL (6 hours)- stimulated hLMVECs, in a concentration-dependent manner The number of migrating MDA-MB-231 cells through the hLMVEC monolayer was quantified by measuring the fluorescence, as described in Methodology. Data represent mean ± SD of three independent experiments. Statistical analysis was performed using one-way ANOVA. Symbols mark the statistical significance levels as follows: (*) indicates p<0.05 as compared to IL-1β 10 ng/mL- stimulated group

In reference to fig. 6b, compound of invention C-3399 inhibited MDA-MB-231 cell transmigration across IL-1β 10 ng/mL (6 hours) -stimulated hLMVECs, in a concentration -dependent manner. The number of migrating MDA-MB-231 cells through the hLMVEC monolayer was quantified by measuring the fluorescence, as described in Methodology. Data represent mean ± SD of three independent experiments. Statistical analysis was performed using one-way ANOVA. Symbols mark the statistical significance levels as follows: (*) indicates p<0.05 as compared to IL-1β 10 ng/mL-stimulated group

Therefore, tested compounds inhibitors of PDIA3 inhibited transmigration of breast cancer cells across hLMVEC monolayer in a concentration -dependent manner.

### Bibliography

Atkin et al. (2008). Neurobiol Dis 30(3):400-407.
Bennett et al. (2000). J. Immunol. 164(8), 4120-4129
Burgess et al. (2000). J. Biol Chem. 275(13):9758-9766;
Cho et al. (2008). J Clin Invest 118(3): 1123-1131.
Colla et al. (2012). J Neurosci 32(10):3306-3320.
Darby and Creighton (1995). Biochemistry. 34(37):11725-11735.
Essex et al. (1999). Br J. Haematol. 104(3):448-454;
Ge et al. (2013). ACS Chem Biol 8(11):2577-2585.
Hashida et al. (2011). J Toxicol Sci 36(1):1-7.
Hatahet et al. (2009). Antioxid Redox Signal. 11(11), 2807-2850
Hayashi et al. (2008). Pflugers Arch. 456:1239-1251.
Hettinghouse A (2018). Pharmacol Ther. 2018 Jan; 181:34-48.
Hotchkiss et al. (1998). Biochim Biophys Acta. 1388(2), 478-488;
Jasuja et al. (2010). Blood 116(22), 4665-4674
Jasuja et al. (2012). J Clin Invest 122(6):2104-2113.
Karala et al. (2010). FEBS J. 277(11), 2454-2462
Khan et al. (2011). ACS Chem. Biol. 6(3), 245-251
Koivu et al. (1987). J Biol Chem 262(14):6447-6449.
Kramkowski et al. (2012). Arterioscler Thromb Vasc Biol. 32:2149-2157.
Lovat et al. (2008). Cancer Res 68(13):5363-5369.
Manickam et al. (2008). Br J Haematol. 140(2), 223-229.
Milczarek et al. (2014). Oncol Rep. 32:491-504.
Reinhardt et al. (2008). J. Clin Invest. 118(3), 1110-1122
Rossowska et al. (2017) Front Immunol.8:713.
Stojak et al., (2018). Pharmacol Res. 136:160-171
Takeda et al. (2019) Chem. Eur. J. 25, 1 - 7
Wetterau et al. (1990). J Biol Chem 265(17):9800-9807.
Xu et al. (2012). Proc Natl Acad Sci USA 109(40):16348-16353.
Yang Z et al. (2018). Onco Targets Ther. 2018;11:4159-4166.
Yoo et al. (2002). Neurosci Lett 334(3):196-200.

## Claims

1. N,N-disubstituted aromatic sulphonamides in form of racemates or enantiomers, or a pharmaceutically acceptable salt, wherein the compounds are chosen from the list:
1-[[5-(Dimethylamino)-1-naphthyl]sulfony1]-N,N-dimethylaziridine-2-carboxamide (C-3371);
N,N-Dimethyl-1-(2-naphthylsulfony1)aziridine-2-carboxamide (C-3373);
Methyl 1-[[6-(dimethylamino)-1-naphthy]]sulfonyl]aziridine-2-carboxylate (C-3375);
Methyl 1-[[6-(dimethylamino)-5-formyl-1-naphthyl]sulfonyl]aziridine-2-carboxylate (C-3376);
1-[[6-(Dimethylamino)-1-naphthyl]sulfony1]-N,N-dimethylaziridine-2-carboxamide (C-3377);
Methyl 1-[[6-(dimethylamino)-5-formyl-2-naphthyl]sulfonyl]aziridine-2-carboxylate (C-3390);
Methyl 1-[[4-(dimethylamino)-1-naphthyl]sulfonyl]aziridine-2-carboxylate (C-3383);
1-[[4-(Dimethylamino)-1-naphthyl]sulfonyl]-N,N-dimethylaziridine-2-carboxamide (C-3385);
1-[[6-(Dimethylamino)-5-formyl-2-naphthy1]sulfony1]-N,N-dimethylaziridine-2-carboxamide (C-3391);
Methyl 1-[[6-(dimethylamino)-2-naphthyl]sulfonyl]aziridine-2-carboxylate (C-3397);
1-[[6-(Dimethylamino)-2-naphthyl]sulfonyl]-N,N-dimethylaziridine-2-carboxamide (C-3398);
Methyl 1-[[5-(dimethylamino)-2-naphthyl]sulfonyl]aziridine-2-carboxylate (C-3399);
1-[[5-(Dimethylamino)-2-naphthyl]sulfonyl]-N,N-dimethylaziridine-2-carboxamide (C-3400);
Methyl 1-[[8-(dimethylamino)-2-naphthyl]sulfonyl]aziridine-2-carboxylate (C-3427);
[1-(4-hexylpheny1)sulfonylaziridin-2-y1]-(1-piperidyl)methanone (C-3520).

2. N,N-disubstituted aromatic sulphonamides or a pharmaceutically acceptable salt selected from the list for use in the treatment or prevention of diseases linked to PDI A3:
1-[[5-(Dimethylamino)-1-naphthyl]sulfonyl]-N,N-dimethylaziridine-2-carboxamide (C-3371);
N,N-Dimethyl-1-(2-naphthylsulfonyl)aziridine-2-carboxamide (C-3373);
Methyl 1-[[6-(dimethylamino)-1-naphthyl]sulfonyl]aziridine-2-carboxylate (C-3375);
Methyl 1-[[6-(dimethylamino)-5-formyl-1-naphthyl]sulfonyl]aziridine-2-carboxylate (C-3376);
1-[[6-(Dimethylamino)-1-naphthyl]sulfonyl]-N,N-dimethylaziridine-2-carboxamide (C-3377);
Methyl 1-[[4-(dimethylamino)-1-naphthyl]sulfonyl]aziridine-2-carboxylate (C-3383);
1-[[4-(Dimethylamino)-1-naphthyl]sulfonyl]-N,N-dimethylaziridine-2-carboxamide (C-3385);
Methyl 1-[[6-(dimethylamino)-5-formyl-2-naphthyl]sulfonyl]aziridine-2-carboxylate (C-3390);
1-[[6-(Dimethylamino)-5-formyl-2-naphthyl]sulfonyl]-N,N-dimethylaziridine-2-carboxamide (C-3391);
Methyl 1-[[6-(dimethylamino)-2-naphthyl]sulfonyl]aziridine-2-carboxylate (C-3397);
1-[[6-(Dimethylamino)-2-naphthyl]sulfonyl]-N,N-dimethylaziridine-2-carboxamide (C-3398);
Methyl 1-[[5-(dimethylamino)-2-naphthyl]sulfonyl]aziridine-2-carboxylate (C-3399);
1-[[5-(Dimethylamino)-2-naphthyl]sulfonyl]-N,N-dimethylaziridine-2-carboxamide (C-3400);
Methyl 1-[[8-(dimethylamino)-2-naphthyl]sulfonyl]aziridine-2-carboxylate (C-3427);
[1-(4-hexylphenyl)sulfonylaziridin-2-yl]-(1-piperidyl)methanone (C-3520);
Methyl (2S)-1-[[6-(dimethylamino)-1-naphthyl]sulfonyl]aziridine-2-carboxylate (C-3548).

3. N,N-disubstituted aromatic sulphonamides for use according to claim 2, in the treatment and prevention of excessive platelet activation and thrombosis, in particular any disease from the list:
disease or condition is thrombosis, thrombotic diseases, in particular the thrombotic disease is acute myocardial infarction, stable angina, unstable angina, acute occlusion following coronary angioplasty and/or stent placement, transient ischemic attacks, cerebrovascular disease, peripheral vascular disease, placental insufficiency, atrial fibrillation, deep vein thrombosis or pulmonary embolism and other pathologies linked with excessive activation of platelets.

4. N,N-disubstituted aromatic sulphonamides for use according to claim 2, in treatment and prevention of cancer in particular any disease from the list:
gastrointestinal cancer, colorectal cancer, colon cancer, liver cancer, hepatocellular carcinoma, pancreatic cancer, biliary tract cancer, stomach cancer, genitourinary cancer, bladder cancer, testicular cancer, cervical cancer, malignant mesothelioma, osteogenic sarcoma, esophageal cancer, laryngeal cancer, prostate cancer, hormone-refractory prostate cancer, lung cancer, small cell lung cancer, non-small cell lung cancer, breast cancer, triple-negative breast cancer, breast cancer having a BRCA1 and/or BRCA2 gene mutation, hematological cancer, leukemia, acute lymphoblastic leukemia, acute myeloid leukemia, chronic lymphocytic leukemia, chronic myeloid leukemia, lymphoma, Hodgkin lymphoma, non-Hodgkin lymphoma, follicular lymphoma, diffuse large B-cell lymphoma, ovarian cancer, brain cancer, neuroblastoma, Ewing's sarcoma, kidney cancer, epidermoid cancer, skin cancer, melanoma, head and/or neck cancer, head and neck squamous cell carcinoma, and mouth cancer.

## Patentansprüche

1. N,N-disubstituierte aromatische Sulfonamide in Form von Racematen oder Enantiomeren oder ein pharmazeutisch akzeptables Salz davon, wobei die Verbindungen aus der folgenden Liste ausgewählt sind:
1-[[5-(Dimethylamino)-1-naphthyl]sulfonyl]-N,N-dimethylaziridin-2-carboxamid (C-3371);
N,N-Dimethyl-1-(2-naphthylsulfonyl)aziridin-2-carboxamid (C-3373);
1-[[6-(Dimethylamino)-1-naphthyl]sulfonyl]aziridin-2-carbonsäuremethylester (C-3375);
1-[[6-(Dimethylamino)-5-formyl-1-naphthyl]sulfonyl]aziridin-2-carbonsäuremethylester (C-3376);
1-[[6-(Dimethylamino)-1-naphthyl]sulfonyl]-N,N-dimethylaziridin-2-carboxamid (C-3377);
1-[[6-(Dimethylamino)-5-formyl-2-naphthyl]sulfonyl]aziridin-2-carbonsäuremethylester (C-3390);
1-[[4-(Dimethylamino)-1-naphthyl]sulfonyl]aziridin-2-carbonsäuremethylester (C-3383);
1-[[4-(Dimethylamino)-1-naphthyl]sulfonyl]-N,N-dimethylaziridin-2-carboxamid (C-3385);
1-[[6-(Dimethylamino)-5-formyl-2-naphthyl]sulfonyl]-N,N-dimethylaziridin-2-carboxamid (C-3391);
1-[[6-(Dimethylamino)-2-naphthyl]sulfonyl]aziridin-2-carbonsäuremethylester (C-3397);
1-[[6-(Dimethylamino)-2-naphthyl]sulfonyl]-N,N-dimethylaziridin-2-carboxamid (C-3398);
1-[[5-(Dimethylamino)-2-naphthyl]sulfonyl]aziridin-2-carbonsäuremethylester (C-3399);
1-[[5-(Dimethylamino)-2-naphthyl]sulfonyl]-N,N-dimethylaziridin-2-carboxamid (C-3400);
1-[[8-(Dimethylamino)-2-naphthyl]sulfonyl]aziridin-2-carbonsäuremethylester (C-3427);
[1-(4-Hexylphenyl)sulfonylaziridin-2-yl]-(1-piperidyl)methanon (C-3520).

2. N,N-disubstituierte aromatische Sulfonamide oder ein pharmazeutisch akzeptables Salz davon, ausgewählt aus der folgenden Liste, zur Verwendung bei der Behandlung oder Vorbeugung von Erkrankungen, die mit PDI A3 in Zusammenhang stehen:
1-[[5-(Dimethylamino)-1-naphthyl]sulfonyl]-N,N-dimethylaziridin-2-carboxamid (C-3371);
N,N-Dimethyl-1-(2-naphthylsulfonyl)aziridin-2-carboxamid (C-3373);
1-[[6-(Dimethylamino)-1-naphthyl]sulfonyl]aziridin-2-carbonsäuremethylester (C-3375);
1-[[6-(Dimethylamino)-5-formyl-1-naphthyl]sulfonyl]aziridin-2-carbonsäuremethylester (C-3376);
1-[[6-(Dimethylamino)-1-naphthyl]sulfonyl]-N,N-dimethylaziridin-2-carboxamid (C-3377);
1-[[4-(Dimethylamino)-1-naphthyl]sulfonyl]aziridin-2-carbonsäuremethylester (C-3383);
1-[[4-(Dimethylamino)-1-naphthyl]sulfonyl]-N,N-dimethylaziridin-2-carboxamid (C-3385);
1-[[6-(Dimethylamino)-5-formyl-2-naphthyl]sulfonyl]aziridin-2-carbonsäuremethylester (C-3390);
1-[[6-(Dimethylamino)-5-formyl-2-naphthyl]sulfonyl]-N,N-dimethylaziridin-2-carboxamid (C-3391);
1-[[6-(Dimethylamino)-2-naphthyl]sulfonyl]aziridin-2-carbonsäuremethylester (C-3397);
1-[[6-(Dimethylamino)-2-naphthyl]sulfonyl]-N,N-dimethylaziridin-2-carboxamid (C-3398);
1-[[5-(Dimethylamino)-2-naphthyl]sulfonyl]aziridin-2-carbonsäuremethylester (C-3399);
1-[[5-(Dimethylamino)-2-naphthyl]sulfonyl]-N,N-dimethylaziridin-2-carboxamid (C-3400);
1-[[8-(Dimethylamino)-2-naphthyl]sulfonyl]aziridin-2-carbonsäuremethylester (C-3427);
[1-(4-Hexylphenyl)sulfonylaziridin-2-yl]-(1-piperidyl)methanon (C-3520);
1-[[6-(Dimethylamino)-1-naphthyl]sulfonyl]aziridin-2-carbonsäuremethylester (2S) (C-3548).

3. N,N-disubstituierte aromatische Sulfonamide zur Verwendung gemäß Anspruch 2 bei der Behandlung und Vorbeugung einer übermäßigen Thrombozytenaktivierung und von Thrombosen, insbesondere einer Erkrankung aus der folgenden Liste:
bei der Erkrankung oder dem Zustand handelt es sich um eine Thrombose oder thrombotische Erkrankungen, wobei die thrombotische Erkrankung insbesondere ein akuter Myokardinfarkt, eine stabile Angina pectoris, eine instabile Angina pectoris, ein akuter Verschluss nach einer Koronarangioplastie und/oder Stentimplantation, transitorische ischämische Attacken, zerebrovaskuläre Erkrankungen, periphere Gefäßerkrankungen, Plazentainsuffizienz, Vorhofflimmern, tiefe Venenthrombose oder Lungenembolie sowie andere mit einer übermäßigen Aktivierung von Thrombozyten verbundene Pathologien ist.

4. N,N-disubstituierte aromatische Sulfonamide zur Verwendung gemäß Anspruch 2 bei der Behandlung und Vorbeugung von Krebs, insbesondere einer Erkrankung aus der folgenden Liste:
Magen-Darm-Krebs, kolorektaler Krebs, Darmkrebs, Leberkrebs, hepatozelluläres Karzinom, Bauchspeicheldrüsenkrebs, Krebs der Gallenwege, Magenkrebs, Krebs des Urogenitaltrakts, Blasenkrebs, Hodenkrebs, Gebärmutterhalskrebs, malignes Mesotheliom, osteogenes Sarkom, Speiseröhrenkrebs, Kehlkopfkrebs, Prostatakrebs, hormonrefraktärer Prostatakrebs, Lungenkrebs, kleinzelliges Lungenkarzinom, nicht-kleinzelliges Lungenkarzinom, Brustkrebs, triple-negativer Brustkrebs, Brustkrebs mit einer Mutation im BRCA1- und/oder BRCA2-Gen, hämatologischer Krebs, Leukämie, akute lymphoblastische Leukämie, akute myeloische Leukämie, chronische lymphatische Leukämie, chronische myeloische Leukämie, Lymphom, Hodgkin-Lymphom, Non-Hodgkin-Lymphom, follikuläres Lymphom, diffuses großzelliges B-Zell-Lymphom, Eierstockkrebs, Hirnkrebs, Neuroblastom, Ewing-Sarkom, Nierenkrebs, Epidermoidkarzinom, Hautkrebs, Melanom, Krebs des Kopfes und/oder Halses, Plattenepithelkarzinom des Kopfes und Halses sowie Mundkrebs.

## Revendications

1. Sulfonamides aromatiques N,N-disubstitués sous forme de racémates ou d'énantiomères, ou sel pharmaceutiquement acceptable de ceux-ci, dans lesquels les composés sont choisis dans la liste suivante :
1-[[5-(Diméthylamino)-1-naphtyl]sulfonyl]-N,N-diméthylaziridine-2-carboxamide (C-3371);
N,N-Diméthyl-1-(2-naphtylsulfonyl)aziridine-2-carboxamide (C-3373);
1-[[6-(diméthylamino)-1-naphtyl]sulfonyl]aziridine-2-carboxylate de méthyle (C-3375);
1-[[6-(diméthylamino)-5-formyl-1-naphtyl]sulfonyl]aziridine-2-carboxylate de méthyle (C-3376);
1-[[6-(Diméthylamino)-1-naphtyl]sulfonyl]-N,N-diméthylaziridine-2-carboxamide (C-3377);
1-[[6-(diméthylamino)-5-formyl-2-naphtyl]sulfonyl]aziridine-2-carboxylate de méthyle (C-3390);
1-[[4-(diméthylamino)-1-naphtyl]sulfonyl]aziridine-2-carboxylate de méthyle (C-3383);
1-[[4-(Diméthylamino)-1-naphtyl]sulfonyl]-N,N-diméthylaziridine-2-carboxamide (C-3385);
1-[[6-(Diméthylamino)-5-formyl-2-naphtyl]sulfonyl]-N,N-diméthylaziridine-2-carboxamide (C-3391);
1-[[6-(diméthylamino)-2-naphtyl]sulfonyl]aziridine-2-carboxylate de méthyle (C-3397);
1-[[6-(Diméthylamino)-2-naphtyl]sulfonyl]-N,N-diméthylaziridine-2-carboxamide (C-3398);
1-[[5-(diméthylamino)-2-naphtyl]sulfonyl]aziridine-2-carboxylate de méthyle (C-3399);
1-[[5-(Diméthylamino)-2-naphtyl]sulfonyl]-N,N-diméthylaziridine-2-carboxamide (C-3400);
1-[[8-(diméthylamino)-2-naphtyl]sulfonyl]aziridine-2-carboxylate de méthyle (C-3427);
[1-(4-hexylphényl)sulfonylaziridin-2-yl]-(1-pipéridyl)méthanone (C-3520).

2. Sulfonamides aromatiques N,N-disubstitués ou sel pharmaceutiquement acceptable de ceux-ci, choisis dans la liste suivante, destinés à être utilisés dans le traitement ou la prévention de maladies liées à la PDI A3 :
1-[[5-(Diméthylamino)-1-naphtyl]sulfonyl]-N,N-diméthylaziridine-2-carboxamide (C-3371);
N,N-Diméthyl-1-(2-naphtylsulfonyl)aziridine-2-carboxamide (C-3373);
1-[[6-(diméthylamino)-1-naphtyl]sulfonyl]aziridine-2-carboxylate de méthyle (C-3375);
1-[[6-(diméthylamino)-5-formyl-1-naphtyl]sulfonyl]aziridine-2-carboxylate de méthyle (C-3376);
1-[[6-(Diméthylamino)-1-naphtyl]sulfonyl]-N,N-diméthylaziridine-2-carboxamide (C-3377);
1-[[4-(diméthylamino)-1-naphtyl]sulfonyl]aziridine-2-carboxylate de méthyle (C-3383);
1-[[4-(Diméthylamino)-1-naphtyl]sulfonyl]-N,N-diméthylaziridine-2-carboxamide (C-3385);
1-[[6-(diméthylamino)-5-formyl-2-naphtyl]sulfonyl]aziridine-2-carboxylate de méthyle (C-3390);
1-[[6-(Diméthylamino)-5-formyl-2-naphtyl]sulfonyl]-N,N-diméthylaziridine-2-carboxamide (C-3391);
1-[[6-(diméthylamino)-2-naphtyl]sulfonyl]aziridine-2-carboxylate de méthyle (C-3397);
1-[[6-(Diméthylamino)-2-naphtyl]sulfonyl]-N,N-diméthylaziridine-2-carboxamide (C-3398);
1-[[5-(diméthylamino)-2-naphtyl]sulfonyl]aziridine-2-carboxylate de méthyle (C-3399);
1-[[5-(Diméthylamino)-2-naphtyl]sulfonyl]-N,N-diméthylaziridine-2-carboxamide (C-3400);
1-[[8-(diméthylamino)-2-naphtyl]sulfonyl]aziridine-2-carboxylate de méthyle (C-3427);
[1-(4-hexylphényl)sulfonylaziridin-2-yl]-(1-pipéridyl)méthanone (C-3520);
1-[[6-(diméthylamino)-1-naphtyl]sulfonyl]aziridine-2-carboxylate de méthyle (2S) (C-3548).

3. Sulfonamides aromatiques N,N-disubstitués destinés à être utilisés selon la revendication 2, dans le traitement et la prévention de l'activation plaquettaire excessive et de la thrombose, en particulier de toute maladie choisie dans la liste suivante :
la maladie ou l'affection est une thrombose ou une maladie thrombotique, en particulier la maladie thrombotique étant un infarctus aigu du myocarde, un angor stable, un angor instable, une occlusion aiguë consécutive à une angioplastie coronaire et/ou à la mise en place d'un stent, des accidents ischémiques transitoires, une maladie cérébrovasculaire, une maladie vasculaire périphérique, une insuffisance placentaire, une fibrillation auriculaire, une thrombose veineuse profonde ou une embolie pulmonaire, ainsi que d'autres pathologies liées à une activation excessive des plaquettes.

4. Sulfonamides aromatiques N,N-disubstitués destinés à être utilisés selon la revendication 2, dans le traitement et la prévention du cancer, en particulier de toute maladie choisie dans la liste suivante :
cancer gastro-intestinal, cancer colorectal, cancer du côlon, cancer du foie, carcinome hépatocellulaire, cancer du pancréas, cancer des voies biliaires, cancer de l'estomac, cancer génito-urinaire, cancer de la vessie, cancer du testicule, cancer du col de l'utérus, mésothéliome malin, sarcome ostéogénique, cancer de l'œsophage, cancer du larynx, cancer de la prostate, cancer de la prostate résistant à l'hormonothérapie, cancer du poumon, cancer du poumon à petites cellules, cancer du poumon non à petites cellules, cancer du sein, cancer du sein triple négatif, cancer du sein présentant une mutation du gène BRCA1 et/ou BRCA2, cancer hématologique, leucémie, leucémie aiguë lymphoblastique, leucémie aiguë myéloïde, leucémie lymphoïde chronique, leucémie myéloïde chronique, lymphome, lymphome de Hodgkin, lymphome non hodgkinien, lymphome folliculaire, lymphome diffus à grandes cellules B, cancer de l'ovaire, cancer du cerveau, neuroblastome, sarcome d'Ewing, cancer du rein, cancer épidermoïde, cancer de la peau, mélanome, cancer de la tête et/ou du cou, carcinome épidermoïde de la tête et du cou, et cancer de la bouche.
